(19) Europäisches Patentamt
European Patent Office
Office européen des brevets

(11) EP 1 221 445 B1

(12) **EUROPEAN PATENT SPECIFICATION**

(45) Date of publication and mention of the grant of the patent:
**20.08.2008 Bulletin 2008/34**

(21) Application number: **00966445.9**

(22) Date of filing: **12.10.2000**

(51) Int Cl.:
*C07D 493/04* (2006.01) *A61K 31/4355* (2006.01)
*A61P 35/00* (2006.01)

(86) International application number:
**PCT/JP2000/007087**

(87) International publication number:
**WO 2001/027115 (19.04.2001 Gazette 2001/16)**

(54) **PENTACYCLIC TAXANE COMPOUNDS**

PENTACYCLISCHE TAXAN-VERBINDUNGEN

COMPOSES PENTACYCLIQUES AU TAXANE

(84) Designated Contracting States:
**AT BE CH CY DE DK ES FI FR GB GR IE IT LI LU MC NL PT SE**

(30) Priority: **15.10.1999 JP 29335699**

(43) Date of publication of application:
**10.07.2002 Bulletin 2002/28**

(73) Proprietor: **Daiichi Sankyo Company, Limited**
**Chuo-ku**
**Tokyo (JP)**

(72) Inventors:
- **SOGA, Tsunehiko**
**Daiichi Sankyo Co., Ltd.Kasai R&D Center**
**Tokyo 134-8630 (JP)**
- **UOTO, Kouichi**
**Daiichi Sankyo Co., Ltd.Kasai R&D Center**
**Tokyo 134-8630 (JP)**
- **TAKEDA, Yasuyuki**
**Daiichi Sankyo Co., Ltd.Kasai R&D Center**
**Tokyo 134-8630 (JP)**

(74) Representative: **Kinzebach, Werner et al**
**Reitstötter, Kinzebach & Partner**
**Patentanwälte**
**Sternwartstrasse 4**
**81679 München (DE)**

(56) References cited:
**EP-A1- 0 826 688** **WO-A-95/13270**
**WO-A-97/46260**

- **H. POUJOL ET AL.: "Taxoides: 7-Déshydroxy-10-acétyldocétaxel et Nouveaux Analogues Préparés à partir des Alcaloides de l'If" TETRAHEDRON, vol. 53, no. 37, 1997, pages 12575-12594, XP002236565**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 221 445 B1

## Description

TECHNICAL FIELD

This invention relates to a taxol derivative which can be administered orally and has an antitumor activity.

BACKGROUND ART

Taxol is a natural substance represented by the following chemical structural formula, which can be obtained in a small amount from a bark or other parts of *Taxus brevifolia.*

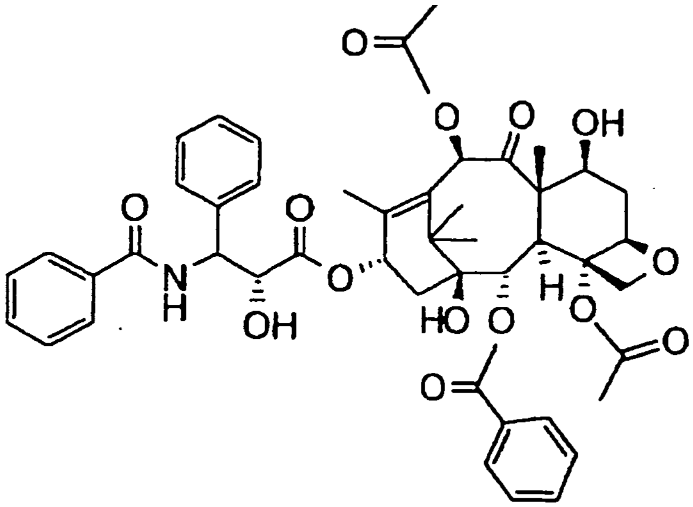

It is known that taxol has an antitumor activity, and its action mechanism is considered to be based on its action to inhibit depolymerization of microtubules in cell division, so that its clinical application is expected as an antitumor agent having an action mechanism which is different from the conventional antitumor agents.

Taxol has so far been obtained from a natural source but only in an extremely small amount. However, taxol derivatives synthesized using a taxol precursor 10-O-deacetylbaccatine III represented by the following formula, which can be obtained from leaves and other parts of Taxus plants in a relatively large amount, have been reported.

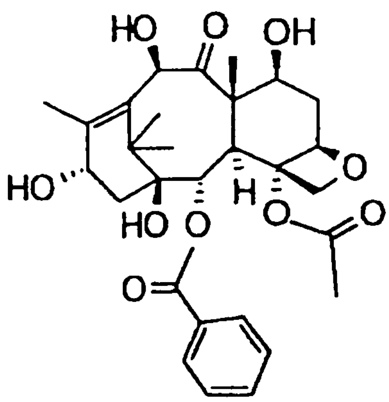

Among them, a compound (taxotere, hereinafter referred to as "compound A") having a structure of the following formula has been drawing attention as a compound having an antitumor activity equal to or higher than that of taxol, and its development as an antitumor agent is in progress.

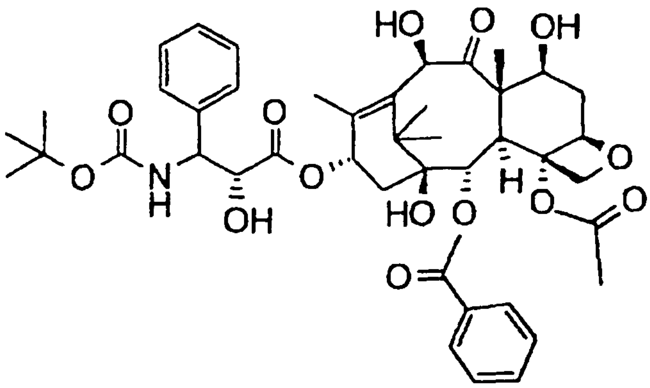

The present inventors have reported that a compound obtained by converting a hydroxyl group formed by reduction of the 9-position ketone and a hydroxyl group of the 10-position into a cyclic acetal form has a strong antitumor activity (JP-A-9-12578, the term "JP-A" as used herein refers to a "published unexamined Japanese patent application").

Taxol, taxotere and the compound disclosed in JP-A-9-12578 are promising as antitumor agents. Regarding the compounds disclosed in Examples of JP-A-9-12578, however, it has a drawback from the toxicity point of view, the efficacy of these compounds by oral administration is not known. From the viewpoint, for example, of lightening the burden on patients at the time of administration and of medical economy, a taxol derivative which can be orally administered is in demand.

As a result of extensive investigation to obtain a taxol derivative which can ensure high safety suited for oral administration while maintaining a high antitumor activity and improving the toxicity problem, the present inventors have conducted extensive studies and obtained a compound (hereinafter, referred to as "compound B") of the following formula capable of showing a significant antitumor activity even by its oral administration for example in an antitumor test using mice.

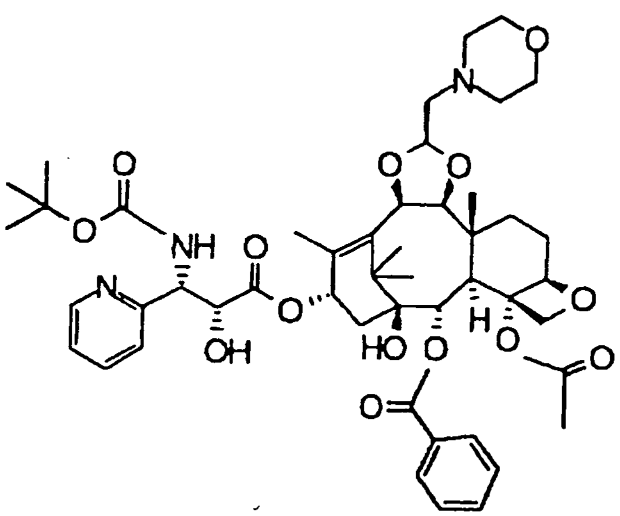

The toxicity problem of this compound was improved in comparison with the compounds disclosed in Examples of JP-A-9-12578. However, its applicability to oral administration in human was not able to be assured, because it was revealed by an *in vitro* metabolism test using human liver microsome that this compound undergoes its metabolism rapidly in human liver microsome.

DISCLOSURE OF INVENTION

With the aim of inhibiting modification of compounds by their metabolism, the inventors have carried out a new

drug design study and found that a compound in which a substituent group is introduced into pyridine ring of the 13-position side chain hardly undergoes its metabolism in human liver microsome and can ensure safety suited for oral administration, while maintaining its antitumor activity and also improving the toxicity problem, thus resulting in the accomplishment of the invention.

Accordingly, the invention provides a compound represented by the following formula or a salt thereof, a medicament which comprises the compound of the following formula or a salt thereof and an antitumor agent which contains the compound of the following formula or a salt thereof.

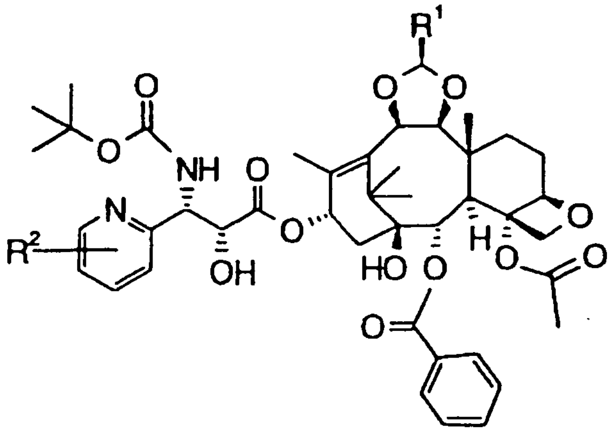

The invention also provides an intermediate (hereinafter, referred to as "intermediate of the invention") represented by the following formula for use in the production of the taxol derivative, and use thereof.

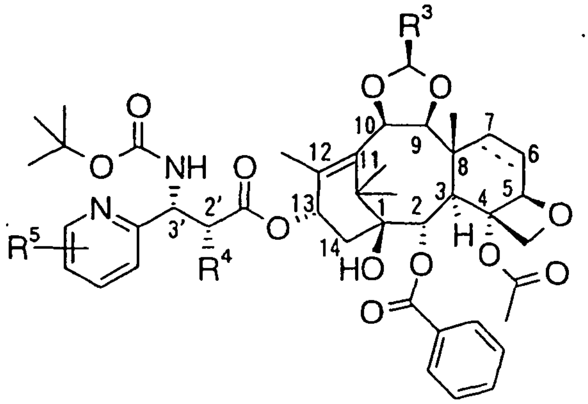

In the following formula, $R^1$ is dimethylaminomethyl group or morpholinomethyl group and $R^2$ is a halogen atom or an alkoxy group having from 1 to 6 carbon atoms. Preferred examples of $R^2$ include methoxy group, fluorine atom and chlorine atom, more preferably fluorine atom and methoxy group.

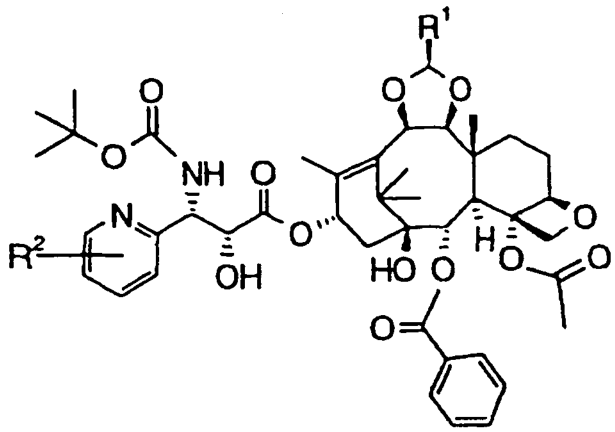

Particularly preferred is a compound represented by the following formula, namely (1S,2S,3R,4S,5R,8R,9S, 10R,13S)-4-acetoxy-2-benzoyloxy-9,10-[(1S)-2-(dimethylamino)ethylidenedioxy]-5,20-epoxy-1-hydroxytax-11-en-13-yl (2R,3S)-3-(tert-butoxycarbonylamino)-3-(3-fluoro-2-pyridyl)-2-hydroxypropionate,

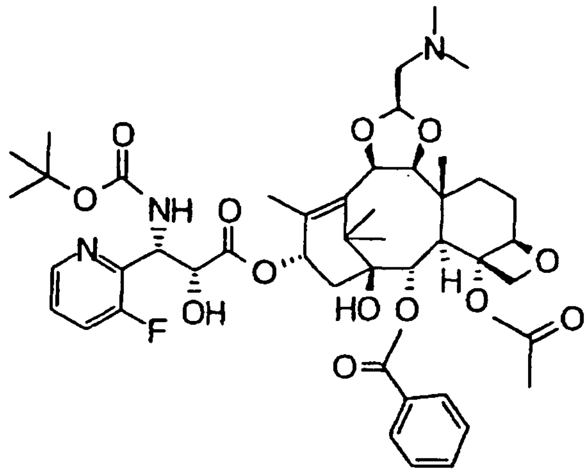

or a salt thereof.

Also in the above-mentioned intermediate of the invention, $R^3$ is dimethylaminomethyl group, morpholinomethyl group or vinyl group, $R^4$ is hydroxyl group which may have a protecting group and $R^5$ is an alkoxy group having from 1 to 6 carbon atoms or a halogen atom. In addition, the part of dotted line between the 6-position and 7-position of a partial structure in the intermediate of the invention, shown by the following formula

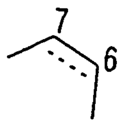

means that the bond of the part may be a double bond.

In the intermediate of the invneiton, examples of the protecting group of $R^4$ include a trialkylsilyl group, benzyl group, a substituted benzyl group, 1-ethoxyethyl group, benzyloxycarbonyl group and 2,2,2-trichloroethoxycarbonyl group. Preferred among them are a trialkylsilyl group such as triisopropylsilyl group, tertiary butyldimethylsilyl group or triethylsilyl group and benzyl group, and particularly preferred are triisopropylsilyl group and benzyl group.

The production intermediate of the taxol derivative of the invention can be used by optionally selecting it in

response to the final product of interest. For example, for the production of a compound of the following formula

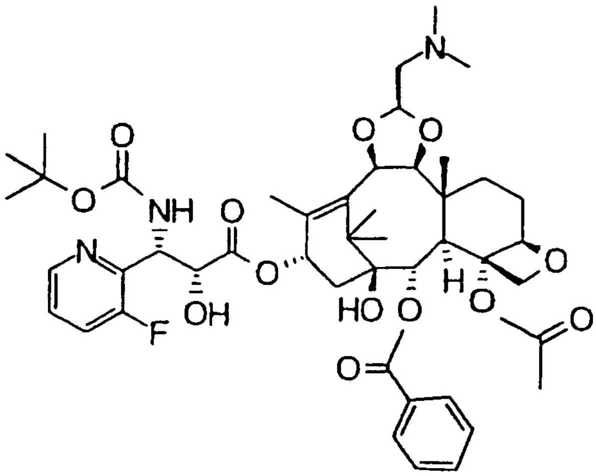

or a salt thereof, it is desirable to use a compound of the following formula

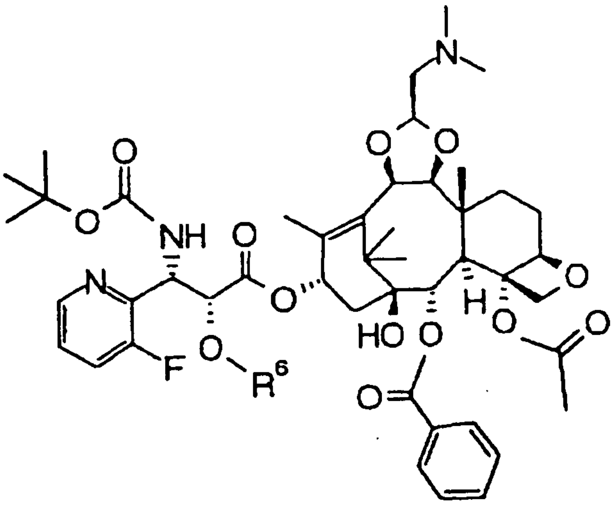

(wherein $R^6$ is triisopropylsilyl group, tertiary butyldimethylsilyl group, triethylsilyl group or benzyl group) or a salt thereof, a compound of the following formula

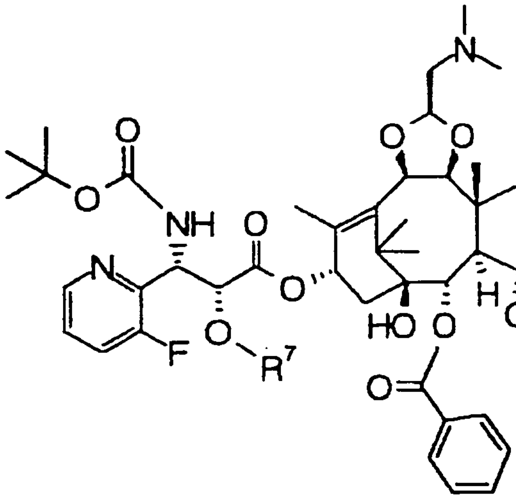

(wherein $R^7$ is triisopropylsilyl group, tertiary butyldimethylsilyl group, triethylsilyl group or benzyl group) or a salt thereof, or a compound of the following formula

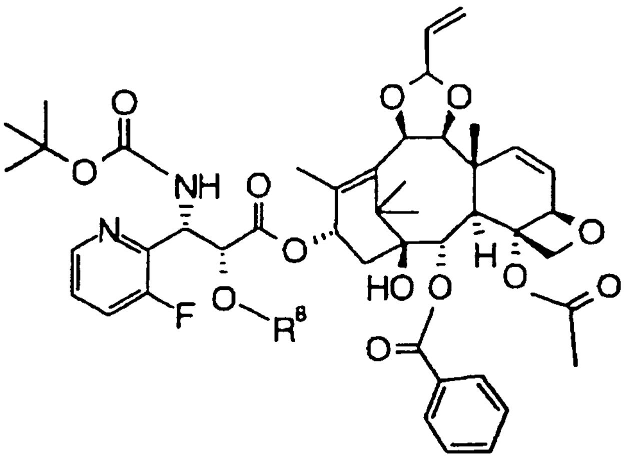

(wherein $R^8$ is triisopropylsilyl group, tertiary butyldimethylsilyl group, triethylsilyl group or benzyl group) or a salt thereof.

The compound of the invention may be in its free form or an acid addition salt. Examples of the acid addition salt include inorganic acid salts such as hydrochloride, sulfate, nitrate, hydrobromide, hydroiodide and phosphate or organic acid salts such as acetate, methane sulfonate, benzenesulfonate, toluenesulfonate, citrate, maleate, fumarate and lactate. It may also be in the form of a solvate, and examples of the solvent include water, methanol, ethanol, propanol, butanol, acetone, acetonitrile, benzene, toluene, tetrahydrofuran and N,N-dimethylformamide.

The compound of the invention can be synthesized in accordance with the method reported in JP-A-9-12578, for example, the following synthetic methods. In this connection, the reaction may be carried out by protecting substituent groups with protecting groups as occasion demands, but the operating order of their deprotection is not particularly limited.

Synthetic Method 1:

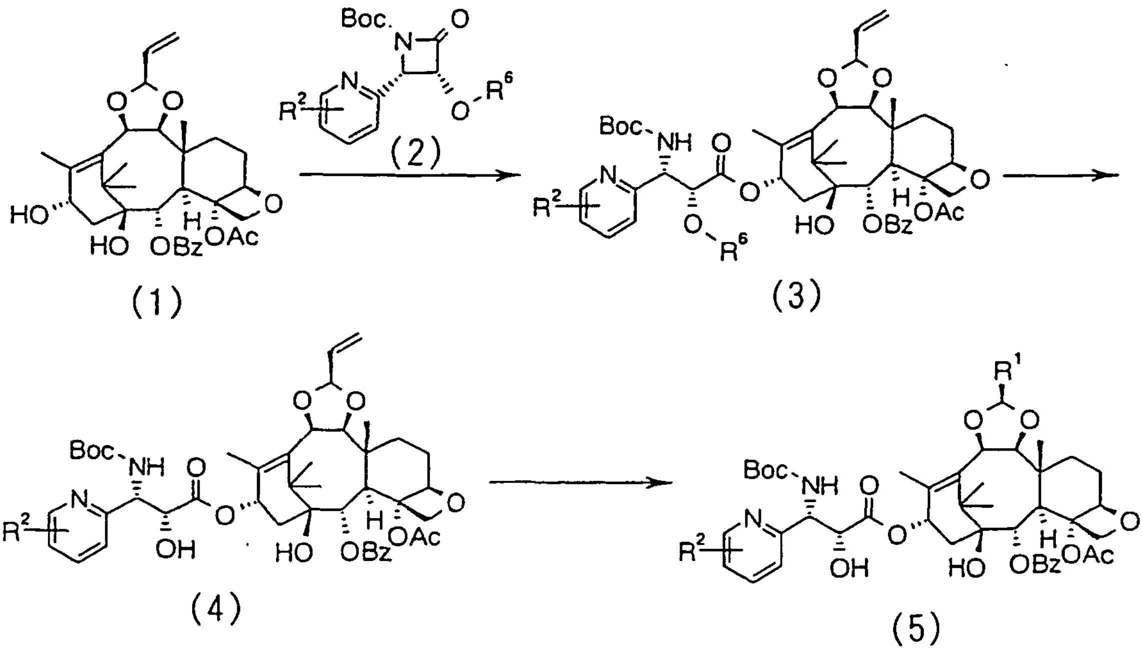

(1) (2) (3)

(4) (5)

A compound (3) is obtained by condensing a compound (1) with a compound (2) in the presence of a base. Then, a protecting group on the hydroxyl group of the thus obtained compound (3) is removed to give a compound (4). The terminal olefin thereof is converted into a diol using an oxidizing agent such as N-methylmorpholine-N-oxide in the presence of an osmium tetraoxide catalyst and then cleaved oxidatively using sodium periodate and the like to form an aldehyde. Thereafter, a reductive reaction with the corresponding amine is carried out to obtain a compound (5).

Synthetic Method 2:

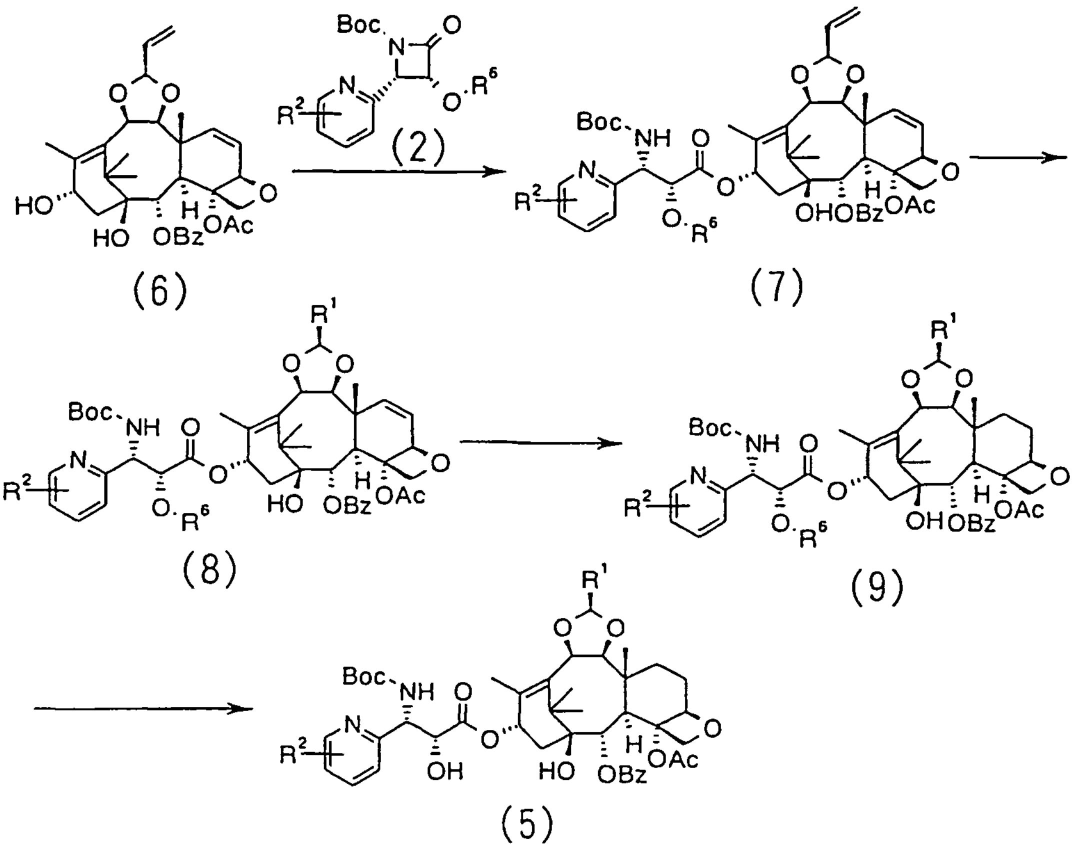

(2)

(6)

(7)

(8)

(9)

(5)

A compound (7) is obtained by condensing a compound (6) with a compound (2) in the same manner as in Synthetic Method 1. Then a compound (8) can be obtained by the conversion of the terminal olefin thereof in the same manner as in Synthetic Method 1. Thereafter, a compound (9) is obtained by reducing the olefin at the 6 and 7-positions by the hydrogenation, and then a protecting group on the hydroxyl group is finally removed, thereby obtaining a compound (5).

The starting materials (1) and (6) in the above Synthetic Methods 1 and 2 can be synthesized in accordance with the method reported in JP-A-9-12578. Also, the compound (2) can be synthesized in accordance with a known synthetic method for β-lactam compound reported by a literature (for example, see J. Am. Chem. Soc. 110, 1917 (1988)).

In the above synthetic methods, $R^1$, $R^2$ and $R^6$ are as defined in the foregoing. Regarding the abbreviations, Boc means tertiary butoxycarbonyl group, Ac means acetyl group and Bz benzoyl group.

In addition, the medicament of the invention can realize treatment of cancers based on its antitumor action, and examples of the object to be treated include various cancers such as lung cancer, gastrointestinal cancer, ovarian cancer, uterine cancer, breast cancer, cancer of liver, cancer of head and neck, blood cancer, renal cancer and testicular tumor.

The compound of the invention can be administered as various injections such as for intravenous injection, intramuscular injection and subcutaneous injection, or by various methods such as oral administration and percutaneous administration. Among these administration methods, oral administration is desirable from the viewpoint of achieving the effects which will be described later. In the case of oral administration, it may be any of free compound or salts.

When a test was carried out using cancer-free mice, the compound of the invention showed no renal toxicity.

Applicability of the compound of the invention as an oral preparation can be predicted by an *in vitro* test which uses human liver microsome. In the case of oral administration, the drug dissolves in the gastrointestinal tract, undergoes its metabolism in the digestive tract and liver and then enters into the blood circulation system. Accordingly, it is considered that metabolism of the drug in the liver exerts influence on the expression of efficacy of the drug. Particularly, it is predicted that the compound of the invention and its analogous compounds undergo their metabolism by CYP3A which is an enzyme distributed in the liver microsome. Thus, prediction of the metabolism by an *in vitro* test using liver microsome is important in considering its clinical use in practice. It has been reported, for example in Pharm. Tech. Japan, 13, 17 - 39, 1997 and J. Pharmacol. Exp. Ther., 283, 46 - 58, 1997, that predicted values of the metabolism by *in vitro* tests

using liver microsome almost coincide with the measured values in human clinical tests. The human liver microsome is available for example from Xenotech LLC, and measurement of the metabolic rate can be carried out making reference to the above journals.

When the drug metabolism rate in liver microsome is measured, bioavailability of the drug can also be calculated as a theoretical value (J. Pharmacol. Exp. Ther., 283, 46 - 58, 1997). Bioavailability is defined as the amount and rate of a drug which reaches the systemic circulating blood, relative to the administered drug (Pharmacokinetic Studies on Drug Development, edited by Yuichi Sugiyama, p. 15, published by Yakuji Jiho). In the case of oral administration, there are various obstacles until a drug enters into the circulating blood, such as dissolution in the gastrointestinal tract, passage through the digestive tract mucous membrane and metabolism in the digestive tract and liver. Thus, it is considered that the range of variability of its final blood concentration, namely bioavailability, among individuals becomes large in comparison with the case of its direct administration into circulating blood. Hellriegel *et al.* have examined bioavailability value and its individual variability (CV value) on 149 articles of various drugs on the market and reported that there is a negative correlation between them (Clin. Pharmacol. Ther., 60, 601 - 607, 1996). That is, it is known that the range of variability of bioavailability among individuals becomes large as the value of bioavailability becomes small.

In the case of antitumor agents, they are administered mostly at around the maximum tolerated dose in order to increase response rate, so that the therapeutic range and the toxicity range draw close to each other and the safety range becomes narrow as the result. Thus, it becomes difficult to use a drug having a large variability range of individual bioavailability as an antitumor agent.

According to the compound of the invention, its metabolic rate in human liver microsome was reduced, and the theoretical value of bioavailability of its unchanged form was also improved. Thus, it was predicted that the variability range of bioavailability values of the unchanged compound among individuals would be small. Because of this effect, it is sufficiently possible to carry out oral administration of the compound of the invention from the safety point of view in enlarging safety range and from viewpoint of effective drug efficacy expression. In this connection, theoretical bioavailability value of the unchanged compound is preferably 0.4 or more, more preferably 0.7 or more.

In addition, applicability of the compound of the invention as oral preparations can also be predicted by a bioavailability (BA) test using monkeys. Metabolism of the compound (B) by the liver microsome of mouse and dog is slow, and its oral absorption property is actually excellent. On the other hand, its metabolism by the monkey liver microsome is quick similar to the case of the human liver microsome. In this case, oral absorption property of the compound (B) in monkey is low. On the contrary, metabolism of the compound of the invention by the monkey liver microsome is slow similar to the case of the mouse and dog liver microsome. Thus, when bioavailability (BA) was measured using monkeys for the purpose of confirming the oral absorption improving effect by the inhibition of metabolism, it was confirmed that the oral absorption in monkey was sharply improved by the compound of the invention in comparison with the compound (B).

Regarding the method for the preparation of pharmaceutical preparations of medicaments and antitumor agents, they can be prepared by selecting appropriate pharmaceutical preparation in response to its administration method and employing a usually used preparation method. Among dosage forms of the antitumor agent of the invention, tablets, powders, granules and capsules can be exemplified as the preparations for oral administration use. Examples of other dosage forms include solutions, syrups, elixirs and oily or aqueous suspensions. Among them, capsules, tablets and solutions are desirable. In the case of injections, additives such as a stabilizer, an antiseptic and a solubilization assisting agent can be used in the preparation. When a solution which may contain such auxiliary substances is made into a solid preparation by freeze-drying or the like means, it can be used as a pharmaceutical preparation which is dissolved before use.

Solutions, suspensions and emulsions can be exemplified as the liquid preparation, and additive agents such as a suspending agent and an emulsifying agent can be used when these pharmaceutical preparations are prepared.

The compound of the invention can be used for the treatment of cancers in mammals, particularly in human, and when administered to human, it is desirable to administer it once a day and repeat it at appropriate intervals.

Regarding its dose, it is desirable to administer it within the range of from about 0.5 mg to 50 mg, preferably from about 1 mg to 20 mg, based on 1 $m^2$ of the body surface area.

The invention is described in detail based on the following examples. In the description of the examples, the following abbreviations will be used. Boc means tertiary butoxycarbonyl group, Ac means acetyl group, Bz means benzoyl group and TIPS means triisopropylsilyl group.

BRIEF DESCRIPTION OF THE DRAWING

Fig. 1 is a graph showing changes with the passage of time in the amount of metabolite formed from respective compounds.

BEST MODE FOR CARRYING OUT INVENTION

(Example 1)

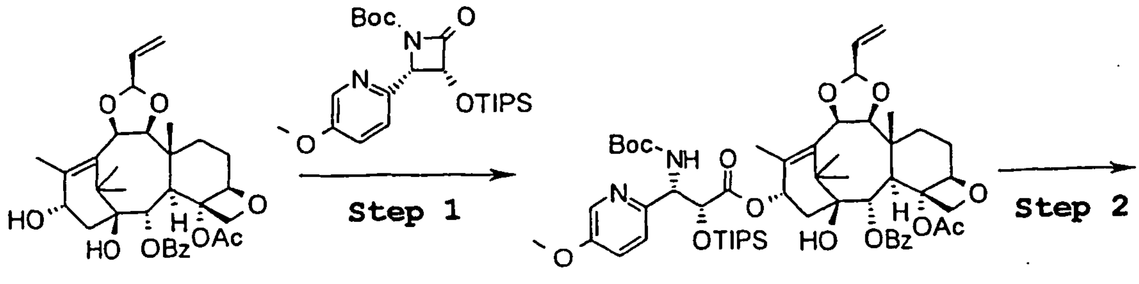


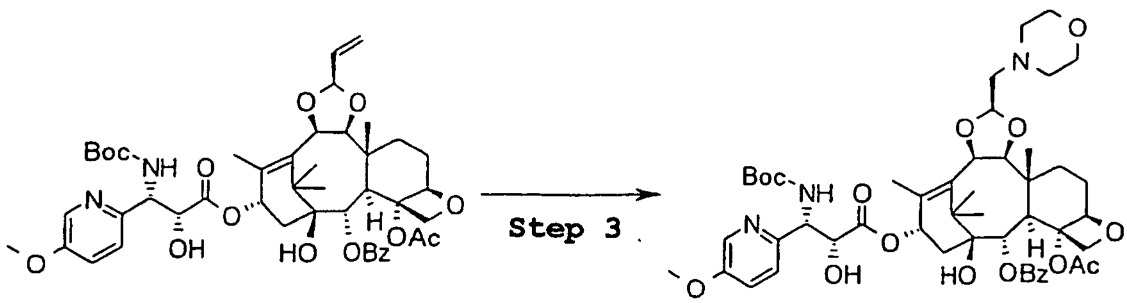


Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(5-methoxy-2-pyridyl)-2-triisopropylsilyloxypropionate

A 300 mg portion of (1S,2S,3R,4S,5R,8R,9S,10R,13S)-4-acetoxy-2-benzoyloxy-5,20-epoxy-1,13-dihydroxy-9,10-(2-propenylidenedioxy)tax-11-ene was dissolved in 10 ml of dry tetrahydrofuran, and the solution was mixed with 0.63 ml of lithium hexamethyldisilazide (1 M tetrahydrofuran solution) at -60°C and stirred for 25 minutes. A 5 ml portion of tetrahydrofuran solution containing 280 mg of (3R,4S)-1-(tert-butoxycarbonyl)-4-(5-methoxy-2-pyridyl)-3-triisopropylsilyloxy-2-azetidinone was added to the reaction solution at the same temperature, and the mixture was stirred under ice-cooling for 40 minutes. Saturated ammonium chloride aqueous solution and ethyl acetate were added to the reaction solution to carry out separation of layers, and the water layer was extracted with ethyl acetate. The organic layers were combined, washed with saturated brine and then dried with anhydrous sodium sulfate. The solvent was evaporated under a reduced pressure and the resulting residue was purified by a silica gel column chromatography (developing solvent; hexane:ethyl acetate = 5:1 (v/v)) to obtain 540 mg of the title compound.
$^1$H-NMR (400 MHz, $CDCl_3$, TMS) δ:
0.89 - 0.95 (21 H, m), 1.32 (3 H, s), 1.33 - 1.62 (3 H, m), 1.41 (9 H, s), 1.52 (3 H, s), 1.65 (3 H, s), 1.82 (3 H, s), 1.92 - 2.32 (3 H, m), 2.49 (3 H, s), 2.98 (1 H, d, J = 4.9 Hz), 3.85 (3 H, s), 4.20 (1 H, d, J = 7.4 Hz), 4.22 (1 H, d, J = 6.8 Hz), 4.32 (1 H, d, J = 8.3 Hz), 4.95 (1 H, s), 5.21 (1 H, d, J = 5.8 Hz), 5.26 - 5.29 (2 H, m), 5.39 - 5.47 (3 H, m), 5.57 (1 H, d, J = 17.6 Hz), 5.96 - 6.02 (2 H, m), 6.11 (1 H, t-like, J = 8.3 Hz), 7.15 (1 H, dd, J = 2.4, 8.8 Hz), 7.31 (1 H, d, J = 8.8 Hz), 7.44 (2 H, t, J = 7.8 Hz), 7.56 (1 H, t, J = 7.8 Hz), 8.13 (2 H, d, J = 7.8 Hz), 8.26 (1 H, d, J = 3.0 Hz).

Step 2: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-2-hydroxy-3-(5-methoxy-2-pyridyl)propionate

A 530 mg portion of the compound obtained in the above step 1 was dissolved in 10 ml of dry tetrahydrofuran, 1.0 ml of tetrabutylammonium fluoride (1M tetrahydrofuran solution) was added to the solution under ice-cooling and

then the mixture was stirred at the same temperature for 30 minutes. Water and ethyl acetate were added to the reaction solution to carry out separation of layers, and the water layer was extracted with ethyl acetate. The organic layers were combined, washed with saturated sodium bicarbonate aqueous solution and saturated brine in that order and then dried using anhydrous sodium sulfate. The solvent was evaporated under a reduced pressure and the resulting residue was purified by a silica gel column chromatography (developing solvent; hexane:ethyl acetate = 1:1 (v/v)) to obtain 410 mg of the title compound.

$^1$H-NMR (400 MHz, $CDCl_3$, TMS) δ:

1.26 (3 H, s), 1.43 (9 H, s), 1.50 (3 H, s), 1.60 - 1.91 (3 H, m), 1.64 (3 H, s), 1.74 (3 H, s), 1.91 (1 H, s), 2.04 - 2.16 (2 H, m), 2.32 - 2.37 (1 H, m), 2.34 (3 H, s), 2.93 (1 H, d, J = 5.3 Hz), 3.85 (3 H, s), 4.18 (1 H, d, J = 7.3 Hz), 4.22 (1 H, d, J = 8.3 Hz), 4.33 (1 H, d, J = 8.3 Hz), 4.79 (1 H, br s), 4.85 (1 H, br s), 4.92 (1 H, br s), 5.23 (1 H, d, J = 5.8 Hz), 5.29 - 5.30 (2 H, m), 5.46 (1 H, d, J = 10.3 Hz), 5.58 (1 H, d, J = 17.1 Hz), 5.90 (1 H, d, J = 9.7 Hz), 5.96 - 6.03 (2 H, m), 6.09 (1 H, t-like, J = 8.4 Hz), 7.22 (1 H, dd, J = 2.4, 8.8 Hz), 7.34 (1 H, d, J = 8.8 Hz), 7.47 (2 H, t, J = 7.8 Hz), 7.60 (1 H, t, J = 7.8 Hz), 8.13 (2 H, d, J = 7.8 Hz), 8.22 (1 H, d, J = 2.4 Hz).

Step 3: (1S,2S,3R,4S,5R,8R,9S,10R,13S)-4-Acetoxy-2-benzoyloxy-5,20-epoxy-1-hydroxy-9,10-[(1S)-2-(morpholino) ethylidenedioxy]tax-11-en-13-yl (2R,3S)-3-(tert-butoxycarbonylamino)-2-hydroxy-3-(5-methoxy-2-pyridyl)propionate

A 400 mg portion of the compound obtained in the above step 2 was dissolved in 5 ml of tetrahydrofuran, and the solution was mixed with 5 ml of acetone, 5 ml of water, 5.9 mg of osmium tetroxide and 270 mg of N-methylmorpholine-N-oxide and stirred at room temperature for 4.5 hours. Ethyl acetate and 10% sodium thiosulfate aqueous solution were added to the reaction solution to carry out separation of layers, and the water layer was extracted with ethyl acetate. The organic layers were combined, washed with saturated sodium bicarbonate aqueous solution and saturated brine in that order and then dried with anhydrous sodium sulfate. The solvent was evaporated under a reduced pressure, the resulting residue was dissolved in 5 ml of tetrahydrofuran and then the solution was mixed with 5 ml of methanol, 5 ml of water and 990 mg of sodium metaperiodate and stirred at room temperature for 1.5 hours. Ethyl acetate and water were added to the reaction solution to carry out separation of layers, and the water layer was extracted with ethyl acetate. The organic layers were combined, washed with saturated brine and then dried with anhydrous sodium sulfate. The solvent was evaporated under a reduced pressure, the resulting residue was dissolved in 30 ml of ethanol and then the solution was mixed with 0.2 ml of morpholine, 0.13 ml of acetic acid and 140 mg of sodium cyanoborohydride and stirred at room temperature for 1 hour. Saturated sodium bicarbonate aqueous solution, ethyl acetate and water were added to the reaction solution to carry out separation of layers, and the water layer was extracted with ethyl acetate. The organic layers were combined, washed with saturated brine and then dried with anhydrous sodium sulfate. The solvent was evaporated under a reduced pressure and the resulting residue was purified by a silica gel column chromatography (developing solvent; chloroform:methanol = 50:1 (v/v)) to obtain 220 mg of the title compound. Melting point: 160 - 161°C

$^1$H-NMR (400 MHz, $CDCl_3$, TMS) δ:

1.27 (3 H, s), 1.43 (9 H, s), 1.48 (3 H, s), 1.60 (3 H, s), 1.72 (3 H, s), 1.78 - 2.12 (6 H, m), 2.31 - 2.38 (1 H, m), 2.34 (3 H, s), 2.58 - 2.68 (4 H, m), 2.71 (1 H, dd, J = 5.4, 13.2 Hz), 2 79 (1 H, dd, J = 3.9, 13.2 Hz), 2.93 (1 H, d, J = 5.3 Hz), 3.75 (4 H, t, J = 4.9 Hz), 3.86 (3 H, s), 4.12 (1 H, d, J = 7.3 Hz), 4.21 (1 H, d, J = 8.3 Hz), 4.33 (1 H, d, J = 8.3 Hz), 4.76 (1 H, br s), 4.85 (1 H, br s), 4.92 (1 H, s), 5.04 (1 H, t, J = 4.6 Hz), 5.23 (1 H, d, J = 6.9 Hz), 5.29 (1 H, d, J = 8.8 Hz), 5.90 (1 H, d, J = 9.3 Hz), 5.98 (1 H, d, J = 4.9 Hz), 6.08 (1 H, t-like, J = 8.3 Hz), 7.22 (1 H, dd, J = 2.9, 8.8 Hz), 7.34 (1 H, d, J = 8.8 Hz), 7.47 (2 H, t, J = 7.8 Hz), 7.60 (1 H, t, J = 7.8 Hz), 8.13 (2 H, d, J = 7.8 Hz), 8.22 (1 H, d, J = 2.9 Hz).

Elemental analysis (for $C_{49}H_{65}N_3O_{15}$)

| | | | |
|---|---|---|---|
| Calcd.: | C, 62.87; | H, 7.00; | N, 4.49 |
| Found : | C, 62.66; x | C, 62.66; H, 7.08; | N, 4.28 |

(Example 2)

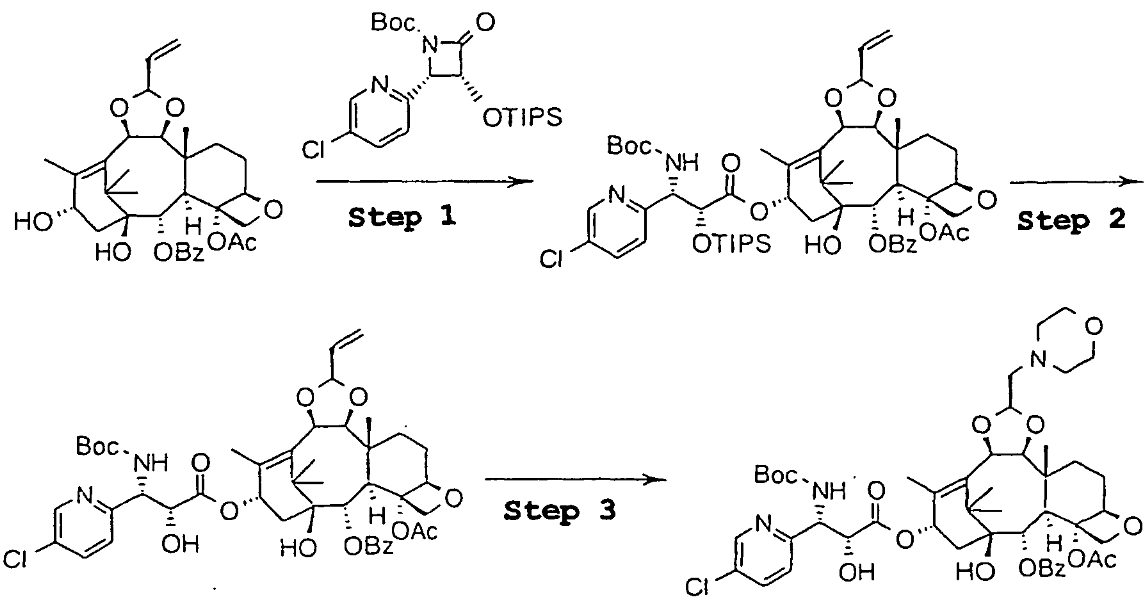


Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(5-chloro-2-pyridyl)-2-triisopropylsilyloxypropionate

The title compound was obtained by repeating the same procedure of the step 1 of Example 1, except that (3R,4S)-1-(tert-butoxycarbonyl)-4-(5-chloro-2-pyridyl)-3-triisopropylsilyloxy-2-azetidinone was used instead of (3R,4S)-1-(tert-butoxycarbonyl)-4-(5-methoxy-2-pyridyl)-3-triisopropylsilyloxy-2-azetidinone.
$^1$H-NMR (400 MHz, $CDCl_3$, TMS) δ:
0.87 - 0.94 (21 H, m), 1.18 - 1.69 (2 H, m), 1.31 (3 H, s), 1.41 (9 H, s), 1.52 (3 H, s), 1.65 (3 H, s), 1.82 (3 H, s), 1.72 - 2.05 (2 H, m), 2.24 - 2.34 (2 H, m), 2.48 (3 H, s), 2.97 (1 H, d, J = 5.4 Hz), 4.19 - 4.23 (2 H, m), 4.33 (1 H, d, J = 7.8 Hz), 4.95 (1 H, s), 5.21 (1 H, d, J = 5.8 Hz), 5.27 - 5.31 (2 H, m), 5.42 - 5.47 (3 H, m), 5.58 (1 H, d, J = 17.5 Hz), 5.96 - 6.04 (2 H, m), 6.11 (1 H, t, J = 8.8 Hz), 7.38 (1 H, d, J = 8.3 Hz), 7.44 (2 H, t, J = 7.3 Hz), 7.57 (1 H, t, J = 7.3 Hz), 7.65 (1 H, dd, J = 8.3 Hz, 2.5 Hz), 8.13 (2 H, d, J = 7.3 Hz), 8.53 (1 H, d, J = 2.5 Hz).

Step 2: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(5-chloro-2-pyridyl)-2-hydroxypropionate

The title compound was obtained by carrying out the same procedure of the step 2 of Example 1, except that the compound obtained in the above step 1 was used as the material.
$^1$H-NMR (400 MHz, $CDCl_3$, TMS) δ:
1.26 (3 H, s), 1.22 - 1.65 (2 H, m), 1.43 (9 H, s), 1.49 (3 H, s), 1.64 (3 H, s), 1.74 (3 H, s), 1.75 - 2.09 (2 H, m), 2.30 - 2.39 (2 H, m), 2.33 (3 H, s), 2.94 (1 H, d, J = 4.9 Hz), 4.18 (1 H, d, J = 5.3 Hz), 4.22 (1 H, d, J = 8.3 Hz), 4.32 (1 H, d, J = 8.3 Hz), 4.61 (1 H, br s), 4.92 (2 H, m), 5.24 (1 H, d, J = 6.3 Hz), 5.30 (1 H, d, J = 6.8 Hz), 5.36 (1 H, d, J = 9.3 Hz), 5.46 (1 H, d, J = 10.5 Hz), 5.58 (1 H, d, J = 17.5 Hz), 5.87 (1 H, d, J = 9.3 Hz), 5.96 - 6.05 (2 H, m), 6.11 (1 H, t, J = 7.8 Hz), 7.39 (1 H, d, J = 8.3 Hz), 7.47 (2 H, t, J = 7.3 Hz), 7.60 (1 H, t, J = 7.3 Hz), 7.69 (1 H, dd, J = 8.3 Hz, 2.4 Hz), 8.12 (2 H, d, J = 7.3 Hz), 8.51 (1 H, d, J = 2.4 Hz).

Step 3: (1S,2S,3R,4S,SR,8R,9S,10R,13S)-4-Acetoxy-2-benzoyloxy-5,20-epoxy-1-hydroxy-9,10-[(1S)-2-(morpholino) ethylidenedioxy]tax-11-en-13-yl (2R,3S)-3-(tert-butoxycarbonylamino)-3-(5-chloro-2-pyridyl)-2-hydroxypropionate

The title compound was obtained by carrying out the same procedure of the step 3 of Example 1, except that the compound obtained in the above step 2 was used as the material.
Melting point: 146 - 150°C
$^1$H-NMR (400 MHz, $CDCl_3$, TMS) δ:
1.26 (3 H, s), 1.20 - 1.72 (2 H, m), 1.43 (9 H, s), 1.48 (3 H, s), 1.63 (3 H, s), 1.73 (3 H, s), 1.75 - 2.03 (2 H, m), 2.33 (3 H, s), 2.30 - 2.38 (2 H, m), 2.59 - 2.69 (4 H, m), 2.72 (1 H, dd, J = 5.4, 13.2 Hz), 2.79 (1 H, dd, J = 3.9, 13.2 Hz), 2.92

(1 H, d, J = 4.9 Hz), 3.74 (4 H, t, J = 4.9 Hz), 4.12 (1 H, d, J = 7.9 Hz), 4.22 (1 H, d, J = 8.8 Hz), 4.32 (1 H, d, J = 8.8 Hz), 4.59 (1 H, br s), 4.91 (2 H, m), 5.05 (1 H, t, J = 4.4 Hz), 5.24 (1 H, d, J = 6.8 Hz), 5.35 (1 H, d, J = 9.3 Hz), 5.87 (1 H, d, J = 9.8 Hz), 5.99 (1 H, d, J = 4.9 Hz), 6.10 (1 H, t, J = 8.0 Hz), 7.39 (1 H, d, J = 8.3 Hz), 7.47 (2 H, t, J = 7.3 Hz), 7.60 (1 H, t, J = 7.3 Hz), 7.69 (1 H, dd, J = 8.3 Hz, 2.4 Hz), 8.12 (2 H, d, J = 7.3 Hz), 8.50 (1 H, d, J = 2.5 Hz).

Elemental analysis (for $C_{48}H_{62}ClN_3O_{14}\cdot H_2O$)

| | | | | |
|---|---|---|---|---|
| Calcd.: | C, 60.15; | H, 6.73; | N, 4.38; | Cl, 3.70 |
| Found : | C, 60.15; | H, 6.74; | N, 4.20; | Cl, 3.63 |

(Example 3)

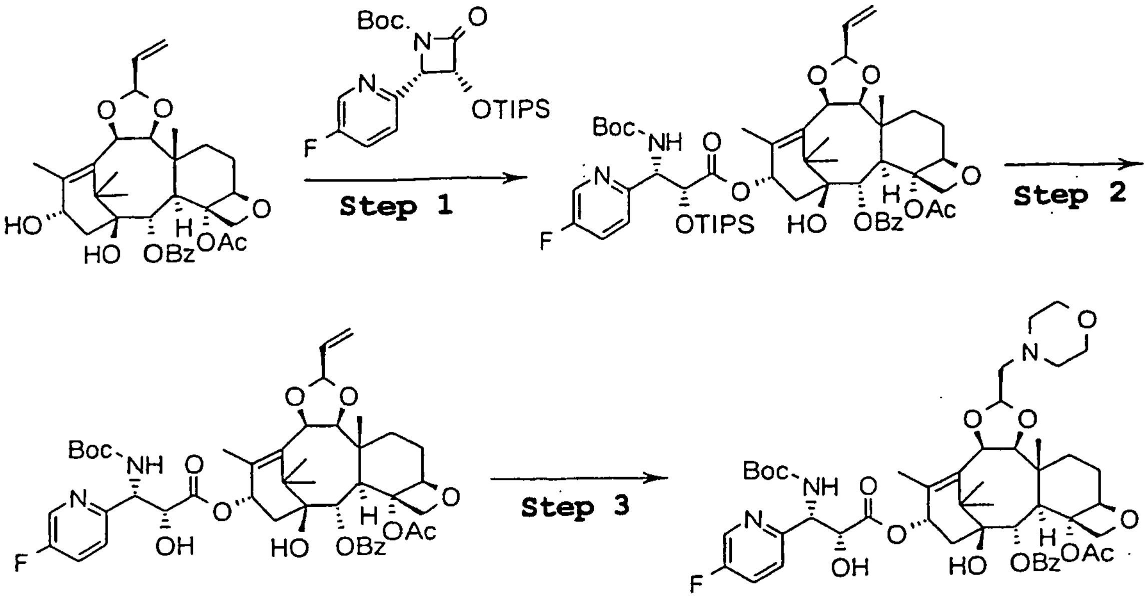


Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(5-fluoro-2-pyridyl)-2-triisopropylsilyloxypropionate

The title compound was obtained by carrying out the same procedure of the step 1 of Example 1, except that (3R,4S)-1-(tert-butoxycarbonyl)-4-(5-fluoro-2-pyridyl)-3-triisopropylsilyloxy-2-azetidinone was used instead of (3R,4S)-1-(tert-butoxycarbonyl)-4-(5-methoxy-2-pyridyl)-3-triisopropylsilyloxy-2-azetidinone.
$^1$H-NMR (400 MHz, $CDCl_3$, TMS) δ:
0.87 - 0.94 (21 H, m), 1.20 - 1.70 (2 H, m), 1.31 (3 H, s), 1.41 (9 H, s), 1.52 (3 H, s), 1.65 (3 H, s), 1.82 (3 H, s), 1.75 - 2.07 (2 H, m), 2.26 - 2.32 (2 H, m), 2.49 (3 H, s), 2.97 (1 H, d, J = 5.4 Hz), 4.19 - 4.23 (2 H, m), 4.33 (1 H, d, J = 8 Hz), 4.96 (1 H, s), 5.21 (1 H, d, J = 5.9 Hz), 5.27 - 5.32 (2 H, m), 5.43 - 5.49 (3 H, m), 5.58 (1 H, d, J = 17.5 Hz), 5.96 - 6.04 (2 H, m), 6.12 (1 H, t, J = 8 Hz), 7.36 - 7.47 (4 H, m) , 7.57 (1 H, t, J = 7.3 Hz), 8.13 (2 H, d, J = 7.3 Hz), 8.43 (1 H, d, J = 2.4 Hz).

Step 2: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(5-fluoro-2-pyridyl)-2-hydroxypropionate

The title compound was obtained by carrying out the same procedure of the step 2 of Example 1, except that the compound obtained in the above step 1 was used as the material.
$^1$H-NMR (400 MHz, $CDCl_3$, TMS) δ:
1.27 (3 H, s), 1.20 - 1.68 (2 H, m) , 1.44 (9 H, s), 1.49 (3 H, s), 1.64 (3 H, s), 1.74 (3 H, s), 1.75 - 2.05 (2 H, m), 2.30 - 2.39 (2 H, m), 2.34 (3 H, s), 2.93 (1 H, d, J = 4.9 Hz), 4.18 (1 H, d, J = 6.8 Hz), 4.23 (1 H, d, J = 8.3 Hz), 4.33 (1 H, d, J = 8.3 Hz), 4.62 (1 H, d, J = 2.5 Hz), 4.90 - 4.92 (2 H, m), 5.24 (1 H, d, J = 5.8 Hz), 5.30 (1 H, d, J = 6.8 Hz), 5.37 (1 H, d, J = 9.3 Hz), 5.46 (1 H, d, J = 10.2 Hz), 5.58 (1 H, d, J = 17 Hz), 5.90 (1 H, d, J = 10.2 Hz), 5.96 - 6.05 (2 H, m), 6.10

(1 H, t, J = 7.8 Hz), 7.40 - 7.49 (4 H, m), 7.60 (1 H, t, J = 7.3 Hz), 8.12 (2 H, d, J = 7.3 Hz), 8.41 (1 H, s).

Step 3: (1S,2S,3R,4S,5R,8R,9S,10R,13S)-4-Acetoxy-2-benzoyloxy-5,20-epoxy-1-hydroxy-9,10-[(1S)-2-(morpholino) ethylidenedioxy]tax-11-en-13-yl (2R,3S)-3-(tert-butoxycarbonylamino)-3-(5-fluoro-2-pyridyl)-2-hydroxypropionate

The title compound was obtained by carrying out the same procedure of the step 3 of Example 1, except that the compound obtained in the above step 2 was used as the material.
Melting point: 148 - 152°C
$^1$H-NMR (400 MHz, $CDCl_3$, TMS) δ:
1.26 (3 H, s), 1.20 - 1.69 (2 H, m), 1.43 (9 H, s), 1.48 (3 H, s), 1.62 (3 H, s), 1.72 (3 H, s), 1.75 - 2.02 (2 H, m), 2.33 (3 H, s), 2.30 - 2.39 (2 H, m), 2.59 - 2.69 (4 H, m), 2.71 (1 H, dd, J = 5.4, 13.2 Hz), 2.79 (1 H, dd, J = 3.9, 13.2 Hz), 2.92 (1 H, d, J = 4.9 Hz), 3.74 (4 H, t, J = 4.9 Hz), 4.12 (1 H, d, J = 7.3 Hz), 4.22 (1 H, d, J = 8.3 Hz), 4.32 (1 H, d, J = 8.3 Hz), 4.60 (1 H, br s), 4.90 - 4.92 (2 H, m), 5.04 (1 H, t, J = 4.9 Hz), 5.24 (1 H, d, J = 6.8 Hz), 5.36 (1 H, d, J = 9.3 Hz), 5.89 (1 H, d, J = 9.8 Hz), 5.99 (1 H, d, J = 4.9 Hz), 6.09 (1 H, t, J = 8.0 Hz), 7.42 - 7.49 (3 H, m), 7.60 (1 H, t, J = 7.3 Hz), 7.60 (1 H, t, J = 7.3 Hz), 8.12 (2 H, d, J = 7.3 Hz), 8.40 (1 H, s).

Elemental analysis (for $C_{48}H_{62}FN_3O_{14}\cdot H_2O$)

| | | | | |
|---|---|---|---|---|
| Calcd.: | C, 61.19; | H, 6.85; | N, 4.46; | F, 2.02 |
| Found : | C, 61.16; | H, 6.85; | N, 4.36; | F, 2.05 |

(Example 4)

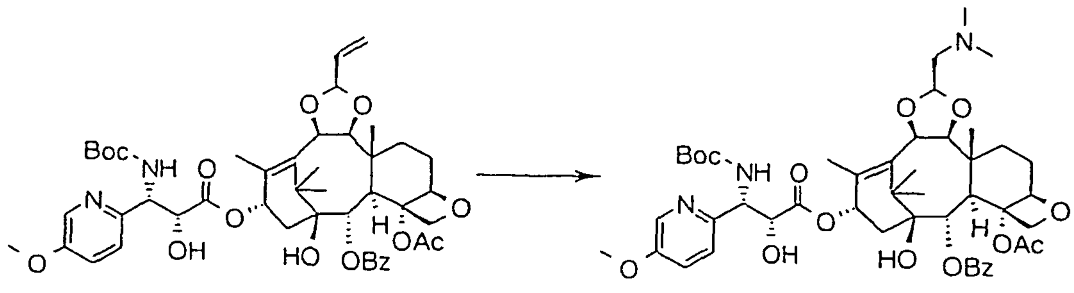

(1S,2S,3R,4S,5R,8R,9S,10R,13S)-4-Acetoxy-2-benzoyloxy-9,10-[(1S)-2-(dimethylamino) ethylidenedioxy]-5,20-epoxy-1-hydroxytax-11-en-13-yl (2R,3S)-3-(tert-butoxycarbonylamino)-2-hydroxy-3-(5-methoxy-2-pyridyl)propionate

The title compound was obtained by carrying out the same procedure of the step 3 of Example 1, except that the compound obtained in the step 2 of Example 1 was used as the material, and dimethylamine (2 M methanol solution) was used instead of morpholine.
$^1$H-NMR (400 MHz, $CDCl_3$, TMS) δ:
1.26 (3 H, s), 1.43 (9 H, s), 1.48 (3 H, s), 1.61 (3 H, s), 1.73 (3 H, s), 1.83 - 1.97 (3 H, m), 2.04 - 2.12 (2 H, m), 2.31 - 2.38 (2 H, m), 2.34 (3 H, s), 2.38 (6 H, s), 2.64 - 2.76 (2 H, m), 2.93 (1 H, d, J = 4.9 Hz), 3.85 (3 H, s), 4.13 (1 H, d, J = 7.4 Hz), 4.21 (1 H, d, J = 8.3 Hz), 4.33 (1 H, d, J = 8.3 Hz), 4.84 (1 H, d, J = 2.4 Hz), 4.92 (1 H, s), 5.01 (1 H, t, J = 4.9 Hz), 5.24 (1 H, d, J = 6.8 Hz), 5.29 (1 H, d, J = 8.8 Hz), 5.91 (1 H, d, J = 9.3 Hz), 5.99 (1 H, d, J = 5.4 Hz), 6.08 (1 H, t, J = 7.8 Hz), 7.23 (1 H, dd, J = 3.0, 8.3 Hz), 7.34 (1 H, d, J = 8.8 Hz), 7.47 (2 H, t, J = 7.8 Hz), 7.60 (1 H, t, J = 7.8 Hz), 8.12 (2 H, d, J = 7.8 Hz), 8.22 (1 H, d, J = 3.0 Hz).

(Example 5)

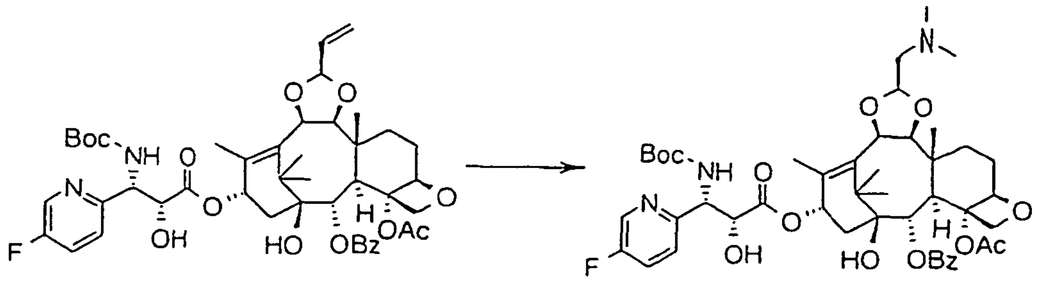

(1S,2S,3R,4S,5R,8R,9S,10R,13S)-4-Acetoxy-2-benzoyloxy-9,10-[(1S)-2-(dimethylamino) ethylidenedioxy]-5,20-epoxy-1-hydroxytax-11-en-13-yl (2R,3S)-3-(tert-butoxycarbonylamino)-3-(5-fluoro-2-pyridyl)-2-hydroxypropionate

The title compound was obtained by carrying out the same procedure of the step 3 of Example 1, except that the compound obtained in the step 2 of Example 3 was used as the material, and dimethylamine (2 M methanol solution) was used instead of morpholine.

$^1$H-NMR (400 MHz, $CDCl_3$, TMS) δ:

1.26 (3 H, s), 1.20 - 1.70 (2 H, m), 1.43 (9 H, s), 1.48 (3 H, s), 1.62 (3 H, s), 1.73 (3 H, s), 1.75 - 2.01 (3 H, m), 2.33 (3 H, s), 2.38 (6 H, s), 2.32 - 2.39 (2 H, m), 2.66 (1 H, dd, J = 5.4, 13.2 Hz), 2.74 (1 H, dd, J = 4.0, 13.2 Hz), 2.93 (1 H, d, J = 4.9 Hz), 4.12 (1 H, d, J = 7.3 Hz), 4.22 (1 H, d, J = 8.3 Hz), 4.32 (1 H, d, J = 8.3 Hz), 4.90 - 4.92 (2 H, m), 5.02 (1 H, t, J = 5.4 Hz), 5.25 (1 H, d, J = 6.8 Hz), 5.36 (1 H, d, J = 6.8 Hz), 5.90 (1 H, d, J = 8.8 Hz), 5.99 (1 H, d, J = 4.9 Hz), 6.09 (1 H, t, J = 8.1 Hz), 7.42 - 7.49 (4 H, m), 7.60 (1 H, t, J = 7.3 Hz), 8.12 (2 H, d, J = 7.3 Hz), 8.41 (1 H, s).

(Example 6)

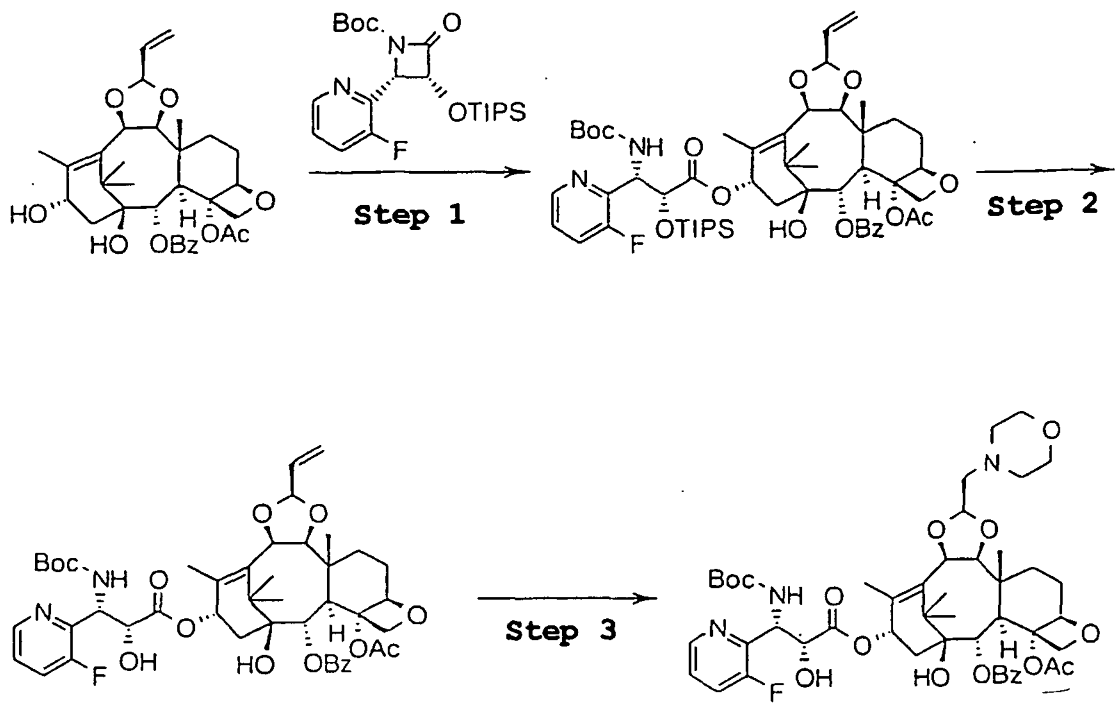


Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(3-fluoro-2-pyridyl)-2-triisopropylsilyloxypropionate

The title compound was obtained by carrying out the same procedure of the step 1 of Example 1, except that

(3R,4S)-1-(tert-butoxycarbonyl)-4-(3-fluoro-2-pyridyl)-3-triisopropylsilyloxy-2-azetidinone was used instead of (3R,4S)-1-(tert-butoxycarbonyl)-4-(5-methoxy-2-pyridyl)-3-triisopropylsilyloxy-2-azetidinone.
$^1$H-NMR (400 MHz, $CDCl_3$, TMS) δ:
0.89 - 0.93 (21 H, m), 1.28 (3 H, s), 1.39 (9 H, s), 1.54 (3 H, s), 1.66 (3 H, s), 1.82 (3 H, s), 1.61 - 1.64 (3 H, m), 1.89 - 1.96 (2 H, m), 2.33 - 2.39 (2 H, m), 2.49 (3 H, s), 2.98 (1 H, d, J = 4.8 Hz), 4.21 - 4.23 (2 H, m), 4.36 (1 H, d, J = 7.8 Hz), 4.96 (2 H, br s), 5.20 (1 H, d, J = 5.9 Hz), 5.27 (1 H, d, J = 6.8 Hz), 5.46 (1 H, d, J = 9.8 Hz), 5.58 (1 H, d, J = 17.1 Hz), 5.61 (1 H, d, J = 6.8 Hz), 5.96 - 6.03 (2 H, m), 6.08 - 6.12 (2 H, m), 7.25 - 7.29 (1 H, m), 7.40 (1 H, t, J = 8.3 Hz), 7.47 (1 H, t, J = 7.8 Hz), 7.59 (1 H, t, J = 7.8 Hz), 8.16 (2 H, d, J = 7.8 Hz), 8.39 (1 H, d, J = 3.4 Hz).

Step 2: (1S,2S,3R,4S,5R,8R,9S,10R,13S)-4-Acetoxy-2-benzoyloxy-5,20-epoxy-1-hydroxy-9,10-(2-propenylidenedioxy)tax-11-en-13-yl (2R,3S)-3-(tert-butoxycarbonylamino)-3-(3-fluoro-2-pyridyl)-2-hydroxypropionate

The title compound was obtained by carrying out the same procedure of the step 2 of Example 1, except that the compound obtained in the above step 1 was used as the material.
$^1$H-NMR (400 MHz, $CDCl_3$, TMS) δ:
1.30 (3 H, s), 1.41 (9 H, s), 1.51 (3 H, s), 1.65 (3 H, s), 1.81 (3 H, s), 1.57 - 1.63 (3 H, m), 1.89 - 1.95 (2 H, m), 2.03 - 2.10 (1 H, m), 2.35 (3 H, s), 2.43 - 2.49 (1 H, m), 2.95 (1 H, d, J = 4.9 Hz), 4.20 (1 H, d, J = 7.4 Hz), 4.23 (1 H, d, J = 8.8 Hz), 4.33 (1 H, d, J = 8.3 Hz), 4.68 (1 H, d, J = 2.5 Hz), 4.92 (1 H, s), 5.24 (1 H, d, J = 6.4 Hz), 5.31 (1 H, d, J = 6.8 Hz), 5.46 (1 H, d, J = 9.8 Hz), 5.58 (1 H, d, J = 17.1 Hz), 5.65 (1 H, d, J = 18.3 Hz), 5.97 - 6.05 (2 H, m), 6.10 (1 H, t, J = 8.8 Hz), 6.21 (1 H, d, J = 8.3 Hz), 7.29 - 7.32 (1 H, m), 7.43 - 7.49 (3 H, m), 7.60 (1 H, t, J = 7.3 Hz), 8.14 (2 H, d, J = 7.3 Hz), 8.41 (1 H, d, J = 4.9 Hz).

Step 3: (1S,2S,3R,4S,5R,8R,9S,10R,13S)-4-Acetoxy-2-benzoyloxy-5,20-epoxy-1-hydroxy-9,10-[(1S)-2-(morpholino) ethylidenedioxy]tax-11-en-13-yl (2R,3S)-3-(tert-butoxycarbonylamino)-3-(3-fluoro-2-pyridyl)-2-hydroxypropionate

The title compound was obtained by carrying out the same procedure of the step 3 of Example 1, except that the compound obtained in the above step 2 was used as the material.
$^1$H-NMR (400 MHz, $CDCl_3$, TMS) δ:
1.29 (3 H, s), 1.40 (9 H, s), 1.49 (3 H, s), 1.61 (3 H, s) , 1.79 (3 H, s), 1.70 - 2.03 (5 H, m), 2.30 - 2.44 (2 H, m), 2.35 (3 H, s), 2.61 - 2.65 (4 H, m), 2.70 - 2.82 (2 H, m), 2.94 (1 H, d, J = 4.8 Hz), 3.75 (4 H, t, J = 4.9 Hz), 4.14 (1 H, d, J = 7.3 Hz), 4.23 (1 H, d, J = 8.3 Hz), 4.33 (1 H, d, J = 7.8 Hz), 4.67 (1 H, s), 4.92 (1 H, s), 5.05 (1 H, t, J = 4.9 Hz), 5.25 (1 H, d, J = 7.3 Hz), 5.65 (1 H, d, J = 7.8 Hz), 5.99 (1 H, d, J = 5.4 Hz), 6.09 (1 H, t, J = 7.8 Hz), 6.20 (1 H, d, J = 8.3 Hz), 7.29 - 7.33 (1 H, m), 7.43 - 7.49 (3 H, m), 7.60 (1 H, t, J = 7.3 Hz), 8.13 (2 H, d, J = 7.3 Hz), 8.40 (1 H, d, J = 4.9 Hz).

(Example 7)

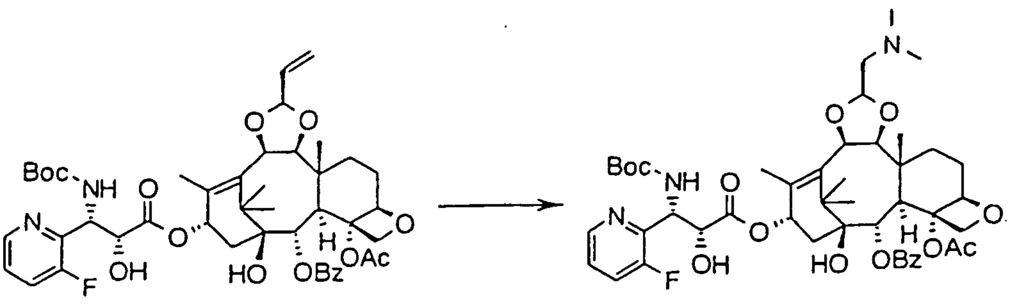

(1S,2S,3R,4S,5R,8R,9S,10R,13S)-4-Acetoxy-2-benzoyloxy-9,10-[(1S)-2-(dimethylamino) ethylidenedioxy]-5,20-epoxy-1-hydroxytax-11-en-13-yl (2R,3S)-3-(tert-butoxycarbonylamino)-3-(3-fluoro-2-pyridyl)-2-hydroxypropionate

The title compound was obtained by carrying out the same procedure of the step 3 of Example 1, except that the compound obtained in the step 2 of Example 6 was used as the material, and dimethylamine (2 M methanol solution) was used instead of morpholine.
$^1$H-NMR (400 MHz, $CDCl_3$, TMS) δ:
1.29 (3 H, s), 1.41 (9 H, s), 1.49 (3 H, s), 1.63 (3 H, s), 1.79 (3 H, s), 1.86 - 2.08 (5 H, m), 2.32 - 2.38 (2 H, m), 2.34 (3 H, s), 2.38 (6 H, s), 2.66 (1 H, dd, J = 5.4, 13.6 Hz), 2.75 (1 H, dd, J = 3.9, 13.6 Hz), 2.94 (1 H, d, J = 4.9 Hz), 4.14 (1

H, d, J = 6.9 Hz), 4.23 (1 H, d, J = 8.3 Hz), 4.33 (1 H, d, J = 8.3 Hz), 4.68 (1 H, d, J = 2.9 Hz), 4.92 (1 H, s), 5.02 (1 H, t, J = 4.9 Hz), 5.25 (1 H, d, J = 6.8 Hz), 5.65 (1 H, d, J = 8.3 Hz), 6.00 (1 H, d, J = 4.9 Hz), 6.09 (1 H, t, J = 7.8 Hz), 6.21 (1 H, d, J = 8.3 Hz), 7.28 - 7.33 (1 H, m), 7.43 - 7.49 (3 H, m), 7.60 (1 H, t, J = 7.3 Hz), 8.14 (2 H, d, J = 7.3 Hz), 8.40 (1 H, d, J = 4.4 Hz).

(Example 8)

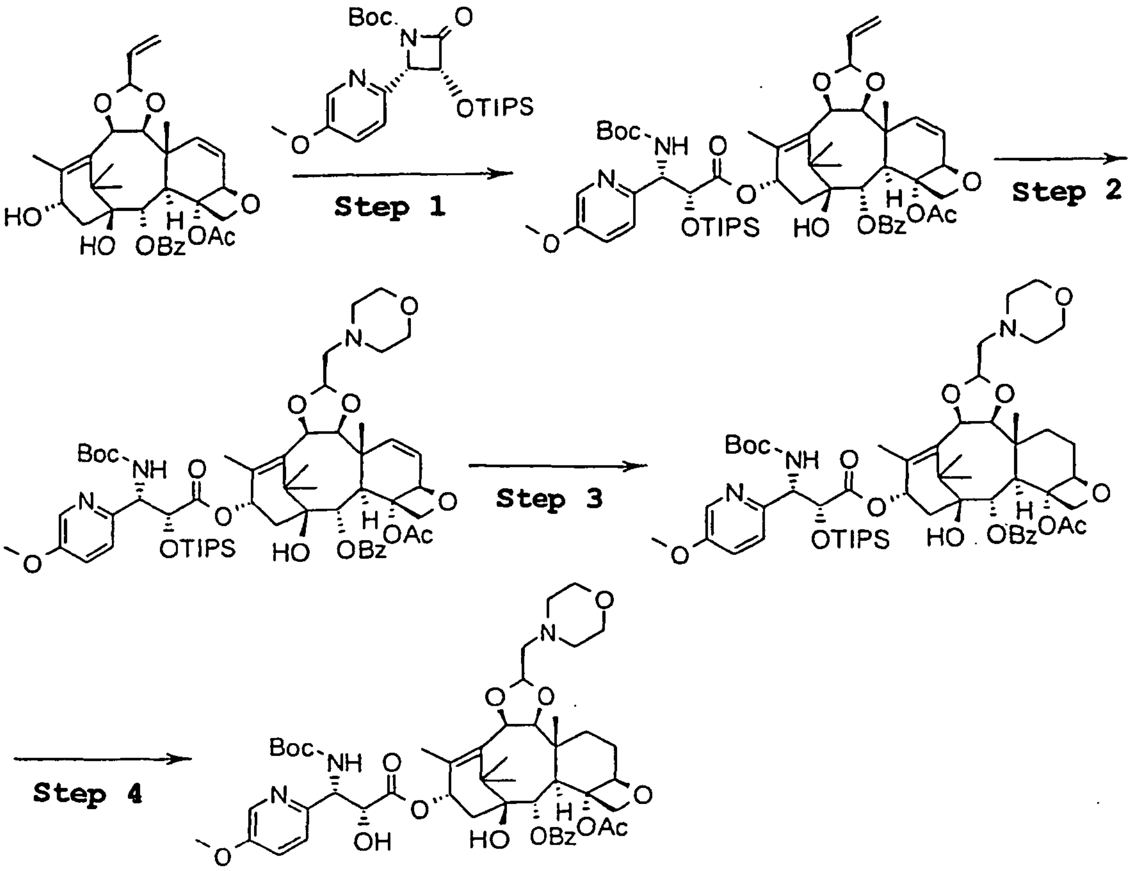


Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-6,11-dien-13-yl (2R,3S)-3-(tert-butoxycarbonylamino)-3-(5-methoxy-2-pyridyl)-2-triisopropylsilyloxypropionate

A 300 mg portion of (1S,2S,3R,4S,5R,8R,9S,10R,13S)-4-acetoxy-2-benzoyloxy-5,20-epoxy-1,13-dihydroxy-9,10-(2-propenylidenedioxy)tax-6,11-diene was dissolved in 10 ml of dry tetrahydrofuran, and the solution was mixed with 0.63 ml of lithium hexamethyldisilazide (1 M tetrahydrofuran solution) at -60°C and stirred for 20 minutes. A 5 ml portion of tetrahydrofuran solution containing 280 mg of (3R,4S)-1-(tert-butoxycarbonyl)-4-(5-methoxy-2-pyridyl)-3-triisopropylsilyloxy-2-azetidinone was added to the reaction solution at the same temperature, and the mixture was stirred under ice-cooling for 30 minutes. Saturated ammonium chloride aqueous solution and ethyl acetate were added to the reaction solution to carry out separation of layers, and the water layer was extracted with ethyl acetate. The organic layers were combined, washed with saturated brine and then dried with anhydrous sodium sulfate. The solvent was evaporated under a reduced pressure and the resulting residue was purified by a silica gel column chromatography (elution with hexane:ethyl acetate = 5:1 (v/v)) to obtain 530 mg of the title compound.
$^1$H-NMR (400 MHz, $CDCl_3$, TMS) δ:
0.87 - 0.93 (21 H, m), 1.29 (3 H, s), 1.41 (9 H, s), 1.54 (3 H, s), 1.69 (3 H, s), 1.75 (3 H, s), 1.82 (1 H, s), 2.29 (1 H, dd, J = 9.8, 15.1 Hz), 2.40 (1 H, dd, J = 8.8, 15.1 Hz), 2.53 (3 H, s), 3.13 (1 H, d, J = 5.8 Hz), 3.85 (3 H, s), 4.04 (1 H, d, J = 7.3 Hz), 4.30 (2 H, br s), 4.90 (1 H, d, J = 3.9 Hz), 5.20 - 5.23 (2 H, m), 5.28 (1 H, d, J = 9.8 Hz), 5.38 (1 H, s), 5.47 - 5.49 (2 H, m), 5.60 (1 H, d, J = 17.0 Hz), 5.71 (1 H, dd, J = 4.4, 10.2 Hz), 5.96 - 6.06 (2 H, m), 6.09 - 6.14 (2 H, m), 7.16 (1 H, dd, J = 2.9, 8.3 Hz), 7.31 (1 H, d, J = 8.3 Hz), 7.47 (2 H, t, J = 7.8 Hz), 7.58 (1 H, t, J = 7.8 Hz), 8.14 (2 H, d, J =

7.8 Hz), 8.26 (1 H, d, J = 2.9 Hz). Step 2: (1S,2S,3R,4S,5R,8R,9S,10R,13S)-4-Acetoxy-2-benzoyloxy-5,20-epoxy-1-hydroxy-9,10-[(1S)-2-(morpholino)ethylidenedioxy]tax-6,11-dien-13-yl (2R,3S)-3-(tert-butoxycarbonylamino)-3-(5-methoxy-2-pyridyl)-2-triisopropylsilyloxy propionate

A 520 mg portion of the compound obtained in the above step 1 was dissolved in 5 ml of tetrahydrofuran, and the solution was mixed with 5 ml of acetone, 5 ml of water, 13 mg of osmium tetraoxide and 300 mg of N-methylmorpholine-N-oxide and stirred at room temperature for 7.5 hours. Ethyl acetate and 10% sodium thiosulfate aqueous solution were added to the reaction solution to carry out separation of layers, and the water layer was extracted with ethyl acetate. The organic layers were combined, washed with saturated sodium bicarbonate aqueous solution and saturated brine in that order and then dried with anhydrous sodium sulfate. The solvent was evaporated under a reduced pressure, the resulting residue was dissolved in 5 ml of tetrahydrofuran and then the solution was mixed with 5 ml of methanol, 5 ml of water and 1.1 g of sodium metaperiodate and stirred at room temperature for 1.5 hours. Ethyl acetate and water were added to the reaction solution to carry out separation of layers, and the water layer was extracted with ethyl acetate. The organic layers were combined, washed with saturated brine and then dried with anhydrous sodium sulfate. The solvent was evaporated under a reduced pressure, the resulting residue was dissolved in 30 ml of ethanol and then the solution was mixed under ice-cooling with 0.22 ml of morpholine, 0.15 ml of acetic acid and 160 mg of sodium cyanoborohydride and stirred at room temperature for 1 hour. Saturated sodium bicarbonate aqueous solution, ethyl acetate and water were added to the reaction solution to carry out separation of layers, and the water layer was extracted with ethyl acetate. The organic layers were combined, washed with saturated brine and then dried with anhydrous sodium sulfate. The solvent was evaporated under a reduced pressure and the resulting residue was purified by a silica gel column chromatography (elution with hexane:ethyl acetate = 3:2 (v/v)) to obtain 290 mg of the title compound.
$^1$H-NMR (400 MHz, $CDCl_3$, TMS) $\delta$:
0.87 - 0.93 (21 H, m), 1.28 (3 H, s), 1.41 (9 H, s), 1.53 (3 H, s), 1.55 (3 H, s), 1.73 (3 H, s), 1.80 (1 H, s), 2.26 (1 H, dd, J = 8.8, 15.1 Hz), 2.39 (1 H, dd, J = 9.8, 15.1 Hz), 2.53 (3 H, s), 2.60 - 2.68 (4 H, m), 2.74 (1 H, dd, J = 4.9, 13.7 Hz), 2.81 (1 H, dd, J = 4.9, 13.7 Hz), 3.12 (1 H, d, J = 5.4 Hz), 3.76 (1 H, t, J = 4.8 Hz), 3.85 (3 H, s), 3.99 (1 H, d, J = 7.9 Hz), 4.30 (2 H, s), 4.89 (1 H, d, J = 3.9 Hz), 5.02 (1 H, t, J = 3.9 Hz), 5.14 (1 H, d, J = 7.3 Hz), 5.27 (1 H, d, J = 9.8 Hz), 5.37 (1 H, d, J = 1.5 Hz), 5.47 (1 H, d, J = 9.8 Hz), 5.69 (1 H, dd, J = 3.9, 10.5 Hz), 5.94 (1 H, d, J = 5.3 Hz), 6.07 - 6.13 (2 H, m), 7.16 (1 H, dd, J = 2.9, 6.3 Hz), 7.30 (1 H, d, J = 6.3 Hz), 7.47 (2 H, t, J = 7.8 Hz), 7.58 (1 H, t, J = 7.8 Hz), 8.15 (2 H, d, J = 7.8 Hz), 8.26 (1 H, d, J = 2.9 Hz).

Step 3: (1S,2S,3R,4S,5R,8R,9S,10R,13S)-4-Acetoxy-2-benzoyloxy-5,20-epoxy-1-hydroxy-9,10-[(1S)-2-(morpholino) ethylidenedioxy]tax-11-en-13-yl (2R,3S)-3-(tert-butoxycarbonylamino)-3-(5-methoxy-2-pyridyl)-2-triisopropylsilyloxypropionate

A 235 mg portion of the compound obtained in the above step 2 was dissolved in 10 ml of ethanol, and the solution was mixed with 235 mg of 5% palladium-carbon catalyst (wet) and shaken for 10 hours under a hydrogen pressure (392 kPa). After removing the catalyst by filtration, the filtrate was concentrated to obtain 230 mg of the title compound.
$^1$H-NMR (400 MHz, $CDCl_3$, TMS) $\delta$:
0.88 - 0.94 (21 H, m), 1.30 (3 H, s), 1.42 (9 H, s), 1.50 (3 H, s), 1.60 (3 H, s), 1.79 (3 H, s), 1.84 - 2.30 (7 H, m), 2.50 (3 H, s), 2.60 - 2.84 (4 H, m), 2.85 - 2.92 (2 H, m), 2.95 (1 H, d, J = 4.4 Hz), 3.80 (4 H, t, J = 4.4 Hz), 3.85 (3 H, s), 4.17 (1 H, d, J = 7.3 Hz), 4.19 (1 H, d, J = 8.7 Hz), 4.33 (1 H, d, J = 8.3 Hz), 4.96 (1 H, s), 5.10 (1 H, br s), 5.22 - 5.28 (2 H, m), 5.40 (1 H, s), 5.48 (1 H, d, J = 10.3 Hz), 5.96 (1 H, d, J = 4.9 Hz), 6.10 (1 H, t, J = 8.3 Hz), 7.12 - 7.17 (1 H, m), 7.31 (1 H, d, J = 8.3 Hz), 7.45 (2 H, t, J = 7.8 Hz), 7.57 (1 H, t, J = 7.8 Hz), 8.13 (2 H, d, J = 7.8 Hz), 8.26 (1 H, d, J = 2.9 Hz).

Step 4: (1S,2S,3R,4S,5R,8R,9S,10R,13S)-4-Acetoxy-2-benzoyloxy-5,20-epoxy-1-hydroxy-9,10-[(1S)-2-(morpholino) ethylidenedioxy]tax-11-en-13-yl (2R,3S)-3-(tert-butoxycarbonylamino)-2-hydroxy-3-(5-methoxy-2-pyridyl)propionate

A 230 mg portion of the compound obtained in the above step 3 was dissolved in 5 ml of dry tetrahydrofuran, 0.43 ml of tetrabutylammonium fluoride (1M tetrahydrofuran solution) was added to the solution under ice-cooling and then the mixture was stirred at the same temperature for 30 minutes. Saturated brine and ethyl acetate were added to the reaction solution to carry out separation of layers, and the water layer was extracted with ethyl acetate. The organic layers were combined, washed with saturated sodium bicarbonate aqueous solution and saturated brine in that order and then dried using anhydrous sodium sulfate. The solvent was evaporated under a reduced pressure and the resulting residue was purified by a silica gel column chromatography (elution with chloroform:methanol = 50:1 (v/v)) and then recrystallized from aqueous ethanol to obtain 110 mg of the title compound. Its instrumental analysis data coincided with those of the compound obtained in the step 3 of Example 1.

(Example 9)

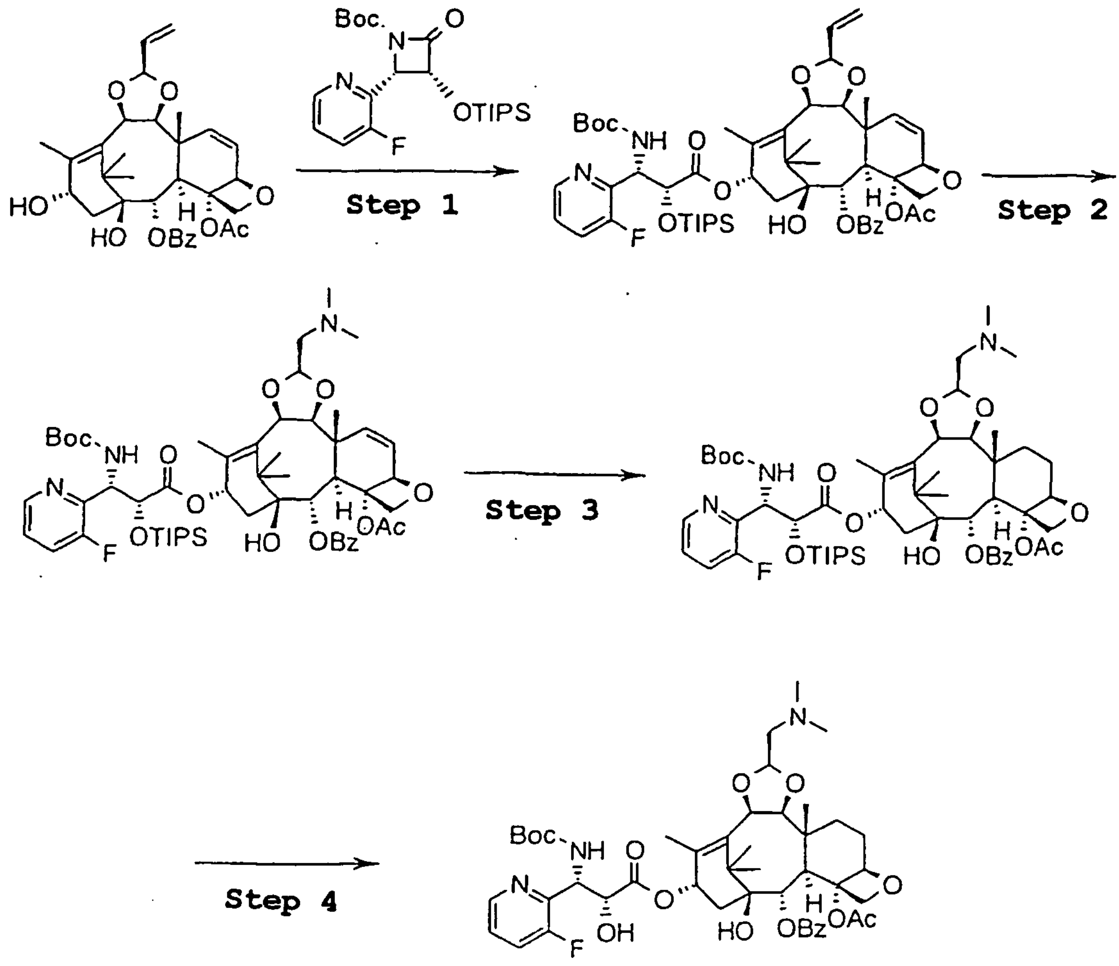


Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-6,11-dien-13-yl (2R,3S)-3-(tert-butoxycarbonylamino)-3-(3-fluoro-2-pyridyl)-2-triisopropylsilyloxypropionate

The title compound was obtained by carrying out the same procedure of the step 1 of Example 8, except that (3R,4S)-1-(tert-butoxycarbonyl)-4-(3-fluoro-2-pyridyl)-3-triisopropylsilyloxy-2-azetidinone was used instead of (3R,4S)-1-(tert-butoxycarbonyl)-4-(5-methoxy-2-pyridyl)-3-triisopropylsilyloxy-2-azetidinone.
$^1$H-NMR (400 MHz, $CDCl_3$, TMS) δ:
0.88 - 0.92 (21 H, m), 1.33 (3 H, s), 1.38 (9 H, s), 1.56 (3 H, s), 1.76 (3 H, s), 2.41 - 2.45 (2 H, m), 2.51 (3 H, s), 3.14 (1 H, d, J = 5.8 Hz), 4.06 (1 H, d, J = 7.8 Hz), 4.33 (2 H, s), 4.90 (1 H, d, J = 4.4 Hz), 4.94 (1 H, d, J = 2.4 Hz), 5.19 - 5.22 (2 H, m), 5.48 (1 H, d, J = 10.3 Hz), 5.58 - 5.64 (2 H, m), 5.70 (1 H, dd, J = 10.3, 4.4 Hz), 5.96 - 6.14 (5 H, m), 7.26 - 7.30 (1 H, m), 7.41 (1 H, t, J = 8.5 Hz), 7.49 (2 H, t, J = 7.5 Hz), 7.59 (1 H, t, J = 7.5 Hz), 8.17 (2 H, d, J = 7.5 Hz), 8.40 (1 H, d, J = 4.4 Hz).

Step 2: (1S,2S,3R,4S,5R,8R,9S,10R,13S)-4-Acetoxy-2-benzoyloxy-9,10-[(1S)-2-(dimethylamino)ethylidenedioxy]-5,20-epoxy-1-hydroxytax-6,11-dien-13-yl (2R,3S)-3-(tert-butoxycarbonylamino)-3-(3-fluoro-2-pyridyl)-2-triisopropylsilyloxypropionate

The title compound was obtained by carrying out the same procedure of the step 2 of Example 8, except that the compound obtained in the above step 1 was used as the material, and dimethylamine (2 M methanol solution) was used instead of morpholine.

$^1$H-NMR (400 MHz, $CDCl_3$, TMS) δ:
0.87 - 0.92 (21 H, m), 1.32 (3 H, s), 1.38 (9 H, s), 1.55 (3 H, s), 1.57 (3 H, s), 1.75 (3 H, s), 2.39 (6 H, s), 2.42 - 2.45 (2 H, m), 2.51 (3 H, s), 2.66 (1 H, dd, J = 5.1, 13.2 Hz), 2.74 (1 H, dd, J = 4.2, 13.2 Hz), 3.14 (1 H, d, J = 5.8 Hz), 4.01 (1 H, d, J = 7.9 Hz), 4.32 (2 H, s), 4.90 - 4.94 (2 H, m), 5.00 (1 H, t, J = 4.9 Hz), 5.15 (1 H, d, J = 7.9 Hz), 5.63 (1 H, d, J = 9.8 Hz), 5.69 (1 H, dd, J = 9.8, 4.4 Hz), 5.95 (1 H, d, J = 5.8 Hz), 6.07 - 6.13 (3 H, m), 7.26 - 7.28 (1 H, m), 7.41 (1 H, t, J = 9.2 Hz), 7.49 (2 H, t, J = 7.5 Hz), 7.59 (1 H, t, J = 7.5 Hz), 8.17 (2 H, d, J = 7.5 Hz), 8.40 (1 H, d, J = 4.4 Hz).

Step 3: (1S,2S,3R,4S,5R,8R,9S,10R,13S)-4-Acetoxy-2-benzoyloxy-9,10-[(1S)-2-(dimethylamino)ethylidenedioxy]-5,20-epoxy-1-hydroxytax-11-en-13-yl (2R,3S)-3-(tert-butoxycarbonylamino)-3-(3-fluoro-2-pyridyl)-2-triisopropylsilyloxypropionate

The title compound was obtained by carrying out the same procedure of the step 3 of Example 8, except that the compound obtained in the above step 2 was used as the material.
$^1$H-NMR (400 MHz, $CDCl_3$, TMS) δ:
0.83 - 0.93 (21 H, m), 1.35 (3 H, s), 1.38 (9 H, s), 1.52 (3 H, s), 1.56 - 2.07 (5 H, m), 1.62 (3 H, s), 1.81 (3 H, s), 2.34 - 2.43 (2 H, m), 2.38 (6 H, s), 2.49 (3 H, s), 2.66 (1 H, dd, J = 5.4, 13.2 Hz), 2.74 (1 H, dd, J = 3.4, 13.2 Hz), 2.98 (1 H, d, J = 5.4 Hz), 4.17 (1 H, d, J = 7.3 Hz), 4.22 (1 H, d, J = 7.8 Hz), 4.36 (1 H, d, J = 8.3 Hz), 4.96 (2 H, s), 5.00 (1 H, t, J = 4.8 Hz), 5.22 (1 H, d, J = 7.3 Hz), 5.60 (1 H, d, J = 8.8 Hz), 5.98 (1 H, d, J = 4.9 Hz), 6.08 - 6.10 (2 H, m), 7.26 - 7.28 (1 H, m), 7.40 (1 H, t, J = 9.2 Hz), 7.48 (2 H, t, J = 7.5 Hz), 7.59 (1 H, t, J = 7.5 Hz), 8.16 (2 H, d, J = 7.5 Hz), 8.40 (1 H, d, J = 3.9 Hz).

Step 4: (1S,2S,3R,4S,5R,8R,9S,10R,13S)-4-Acetoxy-2-benzoyloxy-9,10-[(1S)-2-(dimethylamino)ethylidenedioxy]-5,20-epoxy-1-hydroxytax-11-en-13-yl (2R,3S)-3-(tert-butoxycarbonylamino)-3-(3-fluoro-2-pyridyl)-2-hydroxypropionate

The title compound was obtained by carrying out the same procedure of the step 4 of Example 8, except that the compound obtained in the above step 3 was used as the material. Its instrumental analysis data coincided with those of the compound obtained in the step 3 of Example 7.

(Example 10)

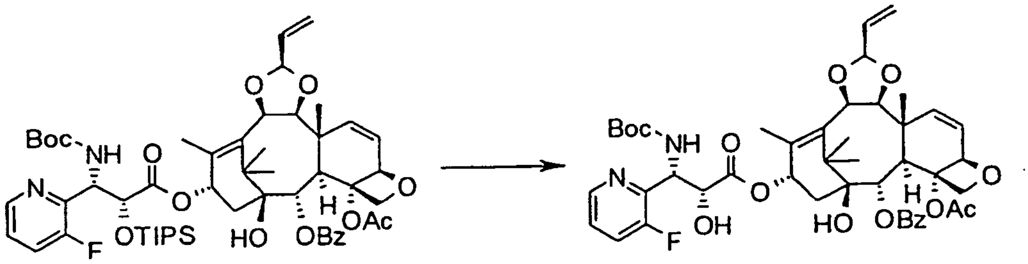

(1S,2S,3R,4S,5R,8R,9S,10R,13S)-4-Acetoxy-2-benzoyloxy-5,20-epoxy-1-hydroxy-9,10-(2-propenylidenedioxy)tax-6,11-dien-13-yl (2R,3S)-3-(tert-butoxycarbonylamino)-3-(3-fluoro-2-pyridyl)-2-hydroxypropionate

The title compound was obtained by carrying out the same procedure of the step 2 of Example 1, except that the compound obtained in the step 1 of Example 9 was used as the material.
$^1$H-NMR (400 MHz, $CDCl_3$, TMS) δ:
1.29 (3 H, s), 1.39 (9 H, s), 1.54 (3 H, s), 1.60 (3 H, s), 1.74 (3 H, s), 1.91 (1 H, s), 2.35 - 2.48 (2 H, m), 2.41 (3 H, s), 3.11 (1 H, d, J = 5.4 Hz), 3.92 (1 H, br s), 4.03 (1 H, d, J = 7.6 Hz), 4.27 (1 H, d, J = 8.1 Hz), 4.33 (1 H, d, J = 8.2 Hz), 4.67 (1 H, br s), 4.87 (1 H, d, J = 4.1 Hz), 5.22 - 5.25 (2 H, m), 5.48 (1 H, d, J = 10.8 Hz), 5.60 (1 H, d, J = 17.3 Hz), 5.62 - 5.64 (1 H, m), 5.69 (1 H, dd, J = 4.1, 10.3 Hz), 5.98 - 6.13 (4 H, m), 6.21 (1 H, d, J = 8.3 Hz), 7.29 - 7.33 (1 H, m), 7.43 - 7.50 (3 H, m), 7.60 (1 H, t, J = 7.3 Hz), 8.15 (2 H, d, J = 7.6 Hz), 8.39 (1 H, d, J = 4.6 Hz) .

(Example 11)

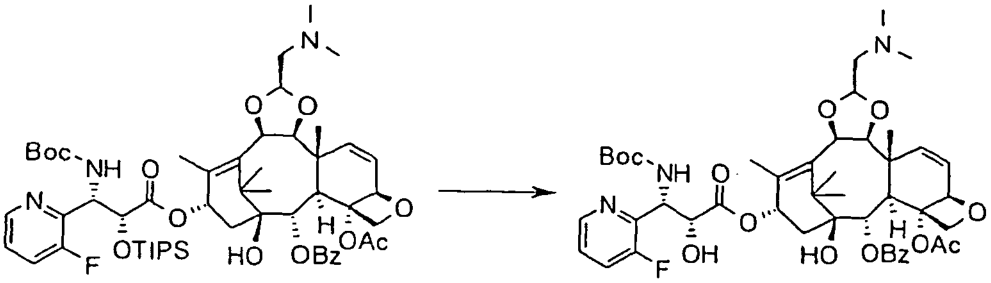

(1S,2S,3R,4S,5R,8R,9S,10R,13S)-4-Acetoxy-2-benzoyloxy-9,10-[(1S)-2-(dimethylamino)ethylidenedioxy]-5,20-epoxy-1-hydroxytax-6,11-dien-13-yl (2R,3S)-3-(tert-butoxycarbonylamino)-3-(3-fluoro-2-pyridyl)-2-hydroxypropionate

The title compound was obtained by carrying out the same procedure of the step 2 of Example 1, except that the compound obtained in the step 2 of Example 9 was used as the material.

$^1$H-NMR (400 MHz, $CDCl_3$, TMS) $\delta$:

1.28 (3 H, s), 1.39 (9 H, s), 1.52 (3 H, s), 1.57 (3 H, s), 1.72 (3 H, s), 1.86 (1 H, s), 2.27 - 2.46 (2 H, m), 2.39 (6 H, s), 2.41 (3 H, s), 2.69 (1 H, dd, J = 5.2, 13.2 Hz), 2.79 (1 H, dd, J = 4.2, 13.2 Hz), 3.11 (1 H, d, J = 5.9 Hz), 3.98 (1 H, d, J = 7.6 Hz), 4.28 (1 H, d, J = 8.1 Hz), 4.33 (1 H, d, J = 8.3 Hz), 4.66 (1 H, d, J = 2.5 Hz), 4.87 (1 H, d, J = 4.1 Hz), 5.02 (1 H, dd, J = 4.2, 4.8 Hz), 5.17 (1 H, d, J = 7.8 Hz), 5.62 (1 H, d, J = 8.5 Hz), 5.68 (1 H, dd, J = 4.1, 10.3 Hz), 5.96 (1 H, m), 6.10 (2 H, m), 6.20 (1 H, d, J = 6.9 Hz), 7.27 - 7.60 (6 H, m), 8.15 (2 H, d, J = 7.3 Hz), 8.40 (1 H, d, J = 4.6 Hz).

(Example 12)

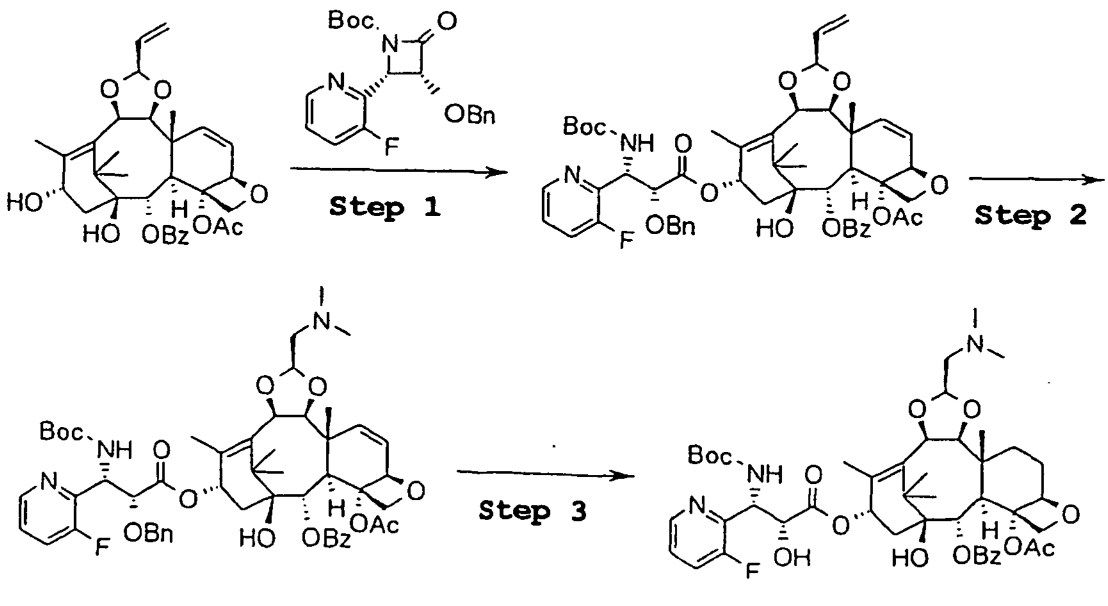

Step 1: (1S,2S,3R,4S,5R,8R,9S,10R,13S)-4-Acetoxy-2-benzoyloxy-5,20-epoxy-1-hydroxy-9,10-(2-propenylidenedioxy)tax-6,11-dien-13-yl (2R,3S)-2-benzyloxy-3-(tert-butoxycarbonylamino)-3-(3-fluoro-2-pyridyl)propionate

The title compound was obtained by carrying out the same procedure of the step 1 of Example 8, except that (3R,4S)-3-benzyloxy-1-(tert-butoxycarbonyl)-4-(3-fluoro-2-pyridyl)-2-azetidinone was used instead of (3R,4S)-1-(tert-butoxycarbonyl)-4-(5-methoxy-2-pyridyl)-3-triisopropylsilyloxy-2-azetidinone.
$^1$H-NMR (400 MHz, $CDCl_3$, TMS) δ:
1.32 (3 H, s), 1.39 (9 H, s), 1.56 (3 H, s), 1.59 (3 H, s), 1.77 (3 H, s), 1.85 (1 H, s), 2.31 (3 H, s)2.39 (2 H, m), 3.13 (1 H, d, J = 6.1 Hz), 4.07 (1 H, d, J = 7.6 Hz), 4.18 (1 H, d, J = 12.0 Hz), 4.31 (3 H, m), 4.68 (1 H, d, J = 12.2 Hz), 4.90 (1 H, d, J = 4.2 Hz), 5.23 (2 H, t, J = 7.1 Hz), 5.48 (1 H, d, J = 11.0 Hz), 5.59 (2 H, m), 5.70 (1 H, dd, J = 4.4, 10.5 Hz), 6.02 (1 H, m), 6.13 (2 H, d, J = 10.2 Hz), 6.26 (1 H, d, J = 9.0 Hz), 6.88 (2 H, d, J = 7.1 Hz), 7.19 (3 H, m), 7.29 (2 H, t, J = 6.8 Hz), 7.49 (2 H, t, J = 7.8 Hz), 7.60 (1 H, t, J = 7.3 Hz), 8.16 (2 H, d, J = 7.3 Hz), 8.42 (1 H, m).

Step 2: (1S,2S,3R,4S,5R,8R,9S,10R,13S)-4-Acetoxy-2-benzoyloxy-9,10-[(1S)-2-(dimethylamino)ethylidenedioxy]-5,20-epoxy-1-hydroxytax-6,11-dien-13-yl (2R,3S)-2-benzyloxy-3-(tert-butoxycarbonylamino)-3-(3-fluoro-2-pyridyl)propionate

The title compound was obtained by carrying out the same procedure of the step 2 of Example 8, except that the compound obtained in the above step 1 was used as the material, and dimethylamine (2 M methanol solution) was used instead of morpholine.
$^1$H-NMR (400 MHz, $CDCl_3$, TMS) δ:
1.26 (3 H, s), 1.39 (9 H, s), 1.54 (3 H, s), 1.57 (3 H, s), 1.75 (3 H, s), 1.82 (1 H, s), 2.31 (3 H, s), 2.36 - 2.39 (2 H, m), 2.38 (6 H, s), 2.71 (1 H, dd, J = 5.2, 13.2 Hz), 2.77 (1 H, dd, J = 4.1, 13.2 Hz), 3.12 (1 H, d, J = 5.6 Hz), 4.02 (1 H, d, J = 7.8 Hz), 4.19 (1 H, d, J = 12.2 Hz), 4.31 (2 H, m), 4.36 (1 H, d, J = 2.9 Hz), 4.68 (1 H, d, J = 12.7 Hz), 4.88 (1 H, d, J = 4.1 Hz), 5.01 (1 H, t, J = 4.7 Hz), 5.16 (1 H, d, J = 7.8 Hz), 5.60 (1 H, d, J = 8.8 Hz), 5.69 (1 H, dd, J = 4.2, 10.3 Hz), 5.93 (1 H, d, J = 5.6 Hz), 6.11 (2 H, m), 6.23 (1 H, d, J = 9.3 Hz), 6.88 (2 H, d, J = 6.6 Hz), 7.16 - 7.31 (5 H, m), 7.48 (1 H, t, J = 7.8 Hz), 7.59 (1 H, t, J = 7.3 Hz), 8.15 (2 H, dd, J = 1.5, 7.1 Hz), 8.41 (1 H, d, J = 2.9 Hz).

Step 3: (1S,2S,3R,4S,5R,8R,9S,10R,13S)-4-Acetoxy-2-benzoyloxy-9,10-[(1S)-2-(dimethylamino)ethylidenedioxy]-5,20-epoxy-1-hydroxytax-11-en-13-yl (2R,3S)-3-(tert-butoxycarbonylamino)-3-(3-fluoro-2-pyridyl)-2-hydroxypropionate

The title compound was obtained by carrying out the same procedure of the step 3 of Example 8, except that the compound obtained in the above step 2 was used as the material. Its instrumental analysis data coincided with those of the compound obtained in the step 3 of Example 7.

(Test Example 1)

Mouse fibrosarcoma Meth A was subcutaneously transplanted into mice (line name; Balb/c), and each compound dissolved in a mixed solvent of ethanol, Tween 80 and 5% glucose (5:5:90 (v/v)) was administered by intravenous injection after 6, 8 or 10 days (or only after 6 days) of the transplantation. Each animal was anatomized on the 17th day to examine tumor weight, platelet numbers and renal toxicity. Six mice were used in each group.
Antitumor effect was calculated by the following formula.

$$\{1 - (\text{tumor weight of compound-administered group}/\text{tumor weight of solvent-administered group})\} \times 100$$

The number of platelets was expressed by (platelets of compound-administered group/platelets of solvent-administered group) x 100.
The renal toxicity findings were expressed as "changed" when a change such as fading was found by macroscopic observation at the time of anatomy or when a change such as precipitation of hyaline droplet substance in the renal tubular cell cytoplasm was found by a histological inspection.

Table 1

| Compound | Dose (mg/kg) | Anti-tumor effect (%) | Platelet numbers (%) | Renal toxicity findings |
|---|---|---|---|---|
| Example 51 of JP-A-9-12578 | 1.5 x 3 | 75 | 37 | changed |
| Example 70 of JP-A-9-12578 | 0.98 x 3 | 78 | 55 | changed |
| Example 81 of JP-A-9-12578 | 2.2 x 3 | 62 | 58 | changed |
| Example 121 of JP-A-9-12578 | 0.43 x 3 | 75 | 57 | changed |
| Compound (B)<br>Compound (B) | 32.4 x 1[a)]<br>22.5 x 1 | 83<br>69 | 142<br>124 | no change |
| a): Three deaths among 6 animals used. | | | | |

(Test Example 2)

B16 Melanoma BL6 was subcutaneously transplanted into mice (C57BL/6), and each compound was administered 4 days thereafter. In the case of intravenous administration, the compound of formula A was administered by dissolving it in a mixed solvent of ethanol, Tween 80 and 5% glucose (5:15:80 (v/v)), and the compound of Example 7 by dissolving in the same mixed solvent of 5:5:90 (v/v). In the case of oral administration, each compound was administered by suspending it in a 0.5% carboxymethylcellulose sodium aqueous solution. Body weight was measured every 2 or 3 days after the administration, and each animal was anatomized after 15 days of the transplantation to measure tumor weight. Antitumor effect was calculated by the following formula.

$${1 - (\text{tumor weight of compound-administered group}/\text{tumor weight of solvent-administered group})} \times 100$$

Six mice were used in each group.

Table 2

| | Dose (mg/kg) | Rout of administration | Antitumor effect (%) |
|---|---|---|---|
| Compound (A) | 180.0 | intravenous | 95.7 |
| | 600.0 | oral | 6.2 |
| Compound (B) | 20.0<br>13.3 | oral<br>oral | 97.4<br>90.5 |
| Compound of Example 7 | 11.9<br>7.9 | intravenous<br>intravenous | 95.5<br>91.5 |
| | 11.9<br>7.9 | oral<br>oral | 97.4<br>91.5 |

(Test Example 3)

Metabolism in human microsome P450

Each of the samples to be evaluated was dissolved in acetonitrile/water (1:1, v/v) to a concentration of 500μM, and the solution was mixed with human liver microsome (Xenotech LLC) and other components such as various coenzymes and buffer solution and allowed to generate the metabolic reaction at 37°C. The reaction solution was

composed of phosphate buffer (0.076 M; final concentration, the same shall apply hereinafter), sample to be evaluated (10 μM), human liver microsome (1 mg/ml), glucose 6-phosphate (10 mM), glucose-6-phosphate dehydrogenase (1 unit/ml), magnesium chloride (4 mM) and reduced nicotinamide adenine dinucleotide phosphate (β-NADPH, 1 mM), and 500 μl of the solution was used in one reaction. In this case, the reaction solution excluding β-NADPH was incubated in advance at 37°C for 2 minutes and then the reaction was started by adding a β-NADPH aqueous solution (50 mM, 10 μl).

The reaction was terminated by adding 1 ml of ice-cooled acetonitrile 1, 2 or 5 minutes after commencement of the reaction.

In this connection, a sample of 0 minute after commencement of the reaction was prepared by adding water instead of the β-NADPH aqueous solution and immediately adding 1 ml of acetonitrile. A 100 μl portion of an internal standard substance was added to each of these samples, and the reaction solution was centrifuged for 15 minutes. The resulting supernatant was injected into a high performance liquid chromatography (HPLC) to measure concentration of the sample to be evaluated. The amount decreased from the concentration at 0 minute of the commencement of reaction was used as the formed amount of the metabolite (nmol/mg protein). By plotting the amount of formed metabolite against the reaction time and carrying out linear regression by the method of least squares, amount of the metabolite formed per 1 minute (metabolic rate constant: k (nmol/min/mg protein)) was calculated from the slope.

From the thus obtained metabolic rate constant k (nmol/min/mg protein), the liver-specific clearance (CLint) was calculated by the following formula.

$$\text{CLint (ml/min/kg body weight)} = \text{k x (g liver weight)/(kg body weight) x (45 mg of microsome protein)/(g liver weight)},$$

wherein the liver weight per 1 kg body weight is 20 g.

Also, the liver clearance (CLh) was calculated from the CLint in accordance with the Well-stirred model (J. Pharmacol. Exp. Ther., 283, 46 - 58, 1997).

$$\text{CLh (ml/min/kg body weight)} = \text{Q x CLint/(Q + CLint)},$$

Theoretical bioavailability (F) value was calculated from the CLh by the following formula.

$$F = (1 - CLh/Q)$$

In addition, theoretical bioavailability value of unchanged compound was calculated by a formula 1 - F.

Table 3

| | Compound (B) | Ex. 1 | Ex. 3 | Ex. 7 |
|---|---|---|---|---|
| Metabolic rate constant (nmol/min/mg protein) | 0.59 | 0.15 | 0.02 | 0.08 |
| Clint (ml/min/kg) | 53.1 | 13.5 | 1.8 | 7.2 |
| Theoretical F value of unchanged compound | 0.27 | 0.60 | 0.92 | 0.74 |

The results are shown in Fig. 1 and Table 1. Theoretical F values of the unchanged form of compounds of the invention were larger that the theoretical F value 0.27 of the unchanged form of the control compound (compound of formula B), meaning that the variability range of bioavailability is suppressed, separation of the therapeutic range and toxicity range can be effected more accurately and the oral administration can therefore be made.

(Test Example 4)

The compound of formula (B) or of Example 7 was intravenously or orally administered to a monkey by single

dose, and changes in its blood concentration was measured to calculate $AUC_{0-\infty}$. The $AUC_{0-\infty}$ means area under the blood concentration time curve of a drug concentration in blood during a period of from the time of administration (0 h) to the infinite time and it can be calculated in accordance with the method (trapezoidal rule) disclosed in Kiyoshi Yamaoka and Yusuke Tanigawara, Yakubutsu Sokudoron Nyumon (A Guide to Pharmacokinetics), pages 116 - 117. In addition, ratio of the AUC at the time of oral administration to the AUC at the time of intravenous administration was calculated as oral BA. Test of the compound of formula (B) was carried out using different individual of one monkey for the intravenous and oral administration, and test of the compound of Example 7 was carried out using the same individuals of 4 monkeys for the intravenous and oral administration to calculate average AUC value.

Animal: female *Macaca irus*, Administration method (compound of formula (B)) [intravenous] EtOH:Tween 80: 5% glucose = 5:5:90, [oral] 0.1 N HCl solution, (compound of Example 7) [intravenous] 10% β-CyD-SBE7 (pH = 3.5 in physiological saline), [oral] 40 mM acetate buffer (pH 4.0)

Table 4

| | | $AUC_{0-\infty}$ (ng·h/ml) | | |
|---|---|---|---|---|
| | Dose (mg/kg) | Intravenous | Oral | Oral BA (%) |
| Compound (B) | 1.6 | 394.1 | 28.1 | 7.1 |
| Compound of Example 7 | 1.8 | 993.8 | 620.6 | 62.4 |

INDUSTRIAL APPLICABILITY

The compound of the invention was improved in terms of its toxicity and showed no renal toxicity. The compound of the invention showed high antitumor effect by its oral administration to mice. Since the compound of the invention has a large theoretical F value of its unchanged form, the variability range of bioavailability is suppressed and separation of the therapeutic range and toxicity range can be effected. The compound of the invention showed excellent oral absorption property in monkey. Accordingly, the compound of the invention can be used as an antitumor agent which can be orally administered.

**Claims**

1. A compound represented by the following formula

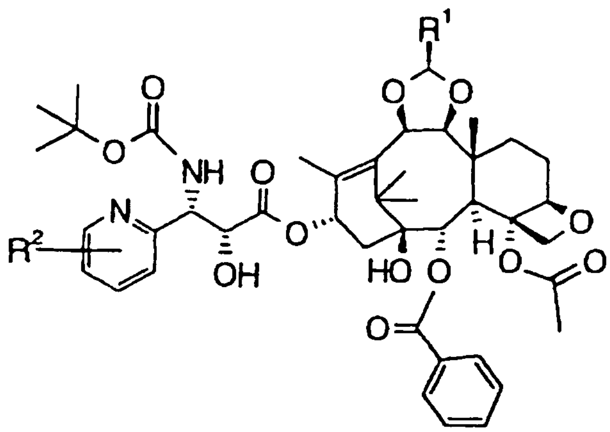

(wherein $R^1$ represents dimethylaminomethyl group or morpholinomethyl group and $R^2$ represents a halogen atom or an alkoxy group having from 1 to 6 carbon atoms) or a salt thereof.

2. The compound or a salt thereof according to claim 1, wherein $R^2$ is methoxy group or fluorine atom.

3. A compound according to claim 1 represented by the following formula

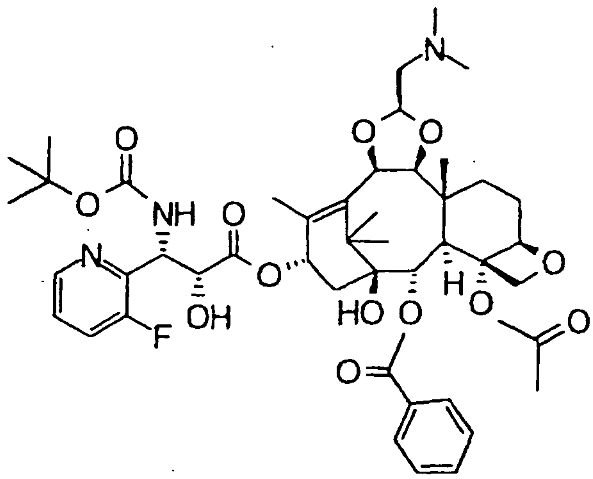

or a salt thereof.

**4.** A medicament which comprises a compound or a salt thereof described in any one of claims 1 to 3.

**5.** An antitumor agent which contains a compound or a salt thereof described in any one of claims 1 to 3.

**6.** A compound represented by the following formula

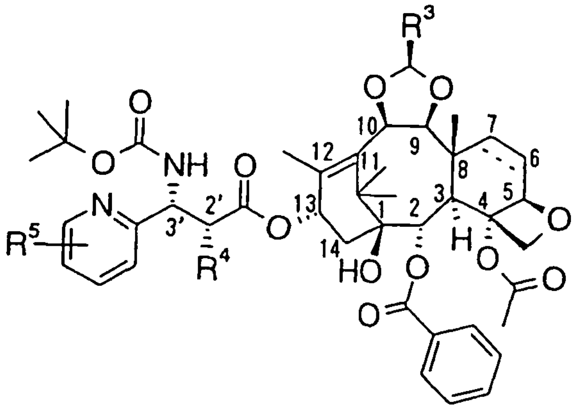

(wherein $R^3$ represents dimethylaminomethyl group, morpholinomethyl group or vinyl group, $R^4$ represents hydroxyl group which may have a protecting group and $R^5$ represents a halogen atom or an alkoxy group having from 1 to 6 carbon atoms, and the part of dotted line between the 6-position and 7-position of a partial structure shown by the following formula

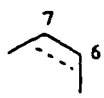

means that the bond of said part may become a double bond) or a salt thereof.

**7.** A compound according to claim 6 represented by the following formula

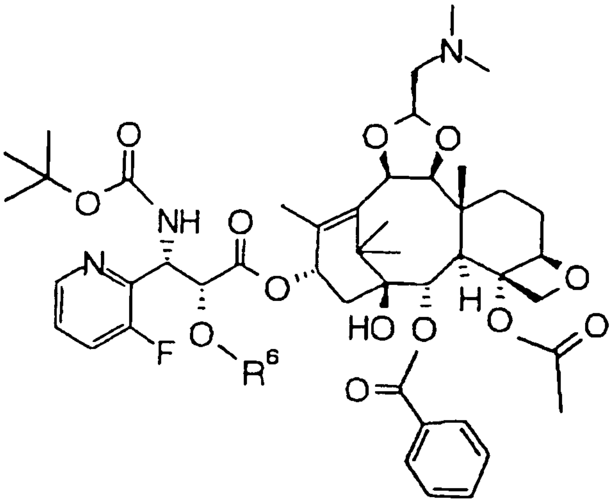

(wherein $R^6$ represents triisopropylsilyl group, tertiary butyldimethylsilyl group, triethylsilyl group or benzyl group) or a salt thereof.

**8.** A compound according to claim 6 represented by the following formula

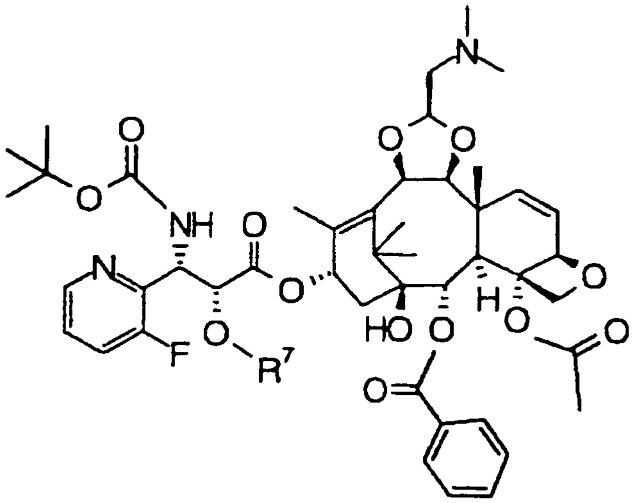

(wherein $R^7$ represents triisopropylsilyl group, tertiary butyldimethylsilyl group, triethylsilyl group or benzyl group) or a salt thereof.

**9.** A compound according to claim 6 represented by the following formula

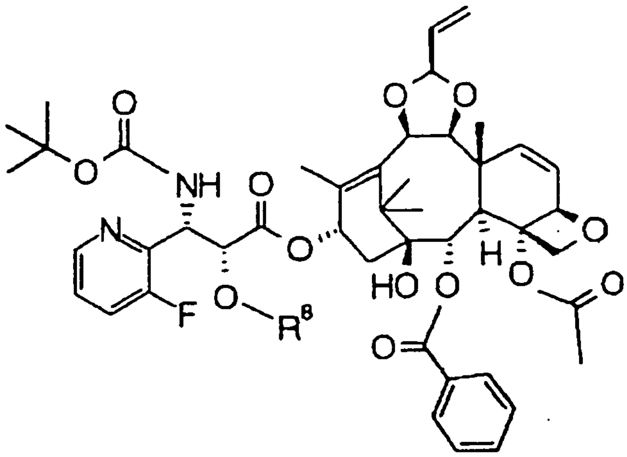

(wherein $R^8$ represents triisopropylsilyl group, tertiary butyldimethylsilyl group, triethylsilyl group or benzyl group) or a salt thereof.

**10.** Use of a compound or a salt thereof described in any one of claims 6 to 9 for producing a compound or a salt thereof described in any one of claims 1 to 3.

**11.** Use according to claim 10 of a compound represented by the following formula

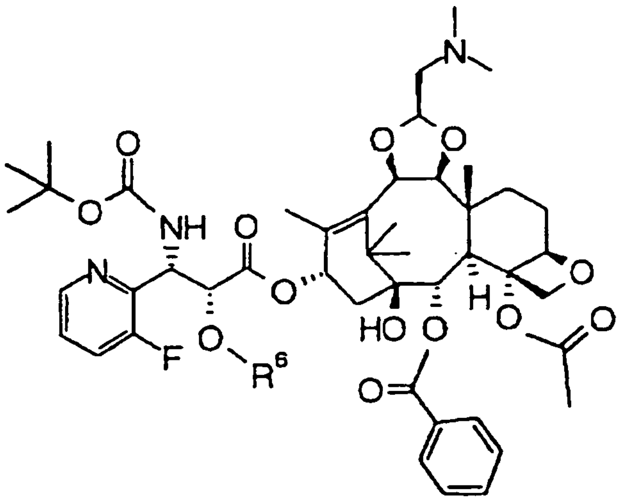

(wherein $R^6$ represents triisopropylsilyl group, tertiary butyldimethylsilyl group, triethylsilyl group or benzyl group) or a salt thereof, for producing a compound represented by the following formula

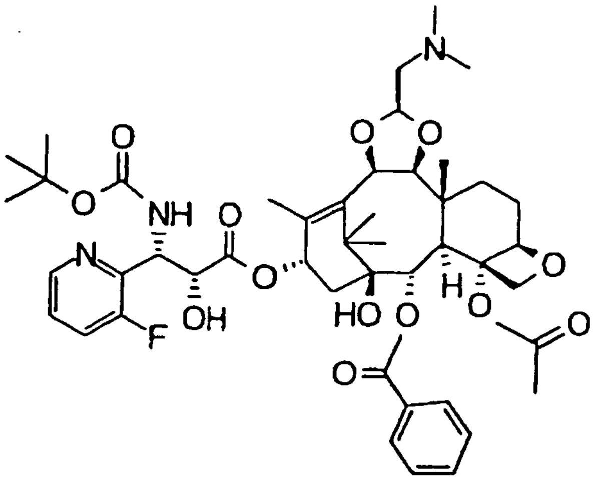

or a salt thereof.

**12.** Use according to claim 10 of a compound represented by the following formula

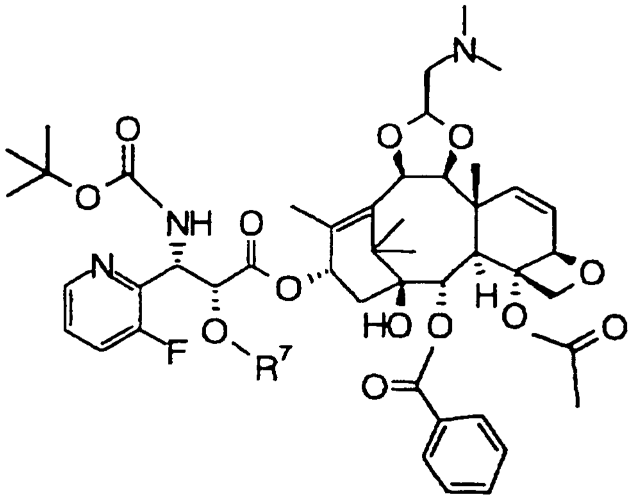

(wherein $R^7$ represents triisopropylsilyl group, tertiary butyldimethylsilyl group, triethylsilyl group or benzyl group) or a salt thereof, for producing a compound represented by the following formula

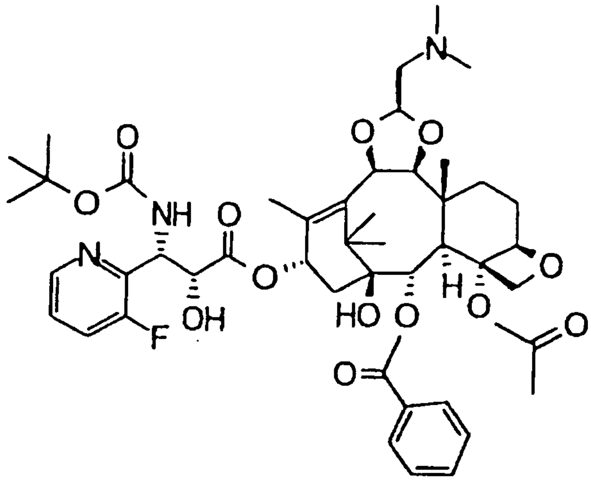

or a salt thereof.

**13.** Use according to claim 10 of a compound represented by the following formula

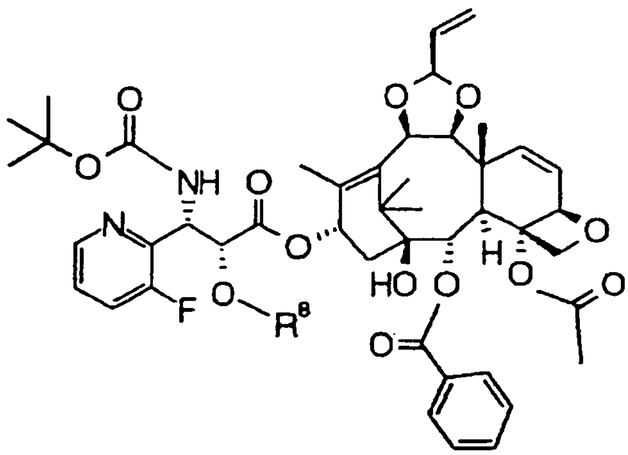

(wherein $R^8$ represents triisopropylsilyl group, tertiary butyldimethylsilyl group, triethylsilyl group or benzyl group) or a salt thereof, for producing a compound represented by the following formula

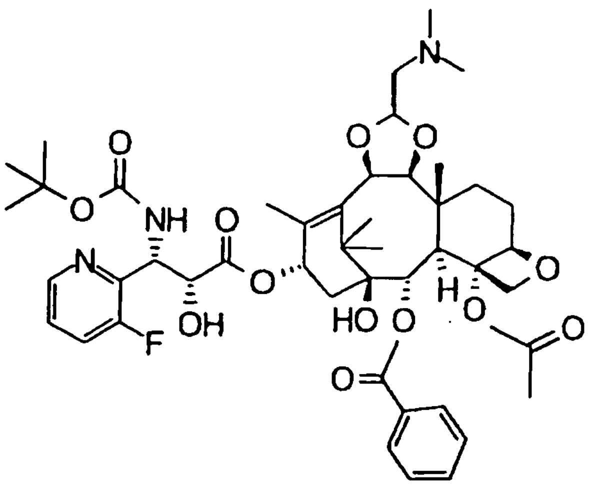

or a salt thereof.

**Patentansprüche**

1. Verbindung der folgenden Formel

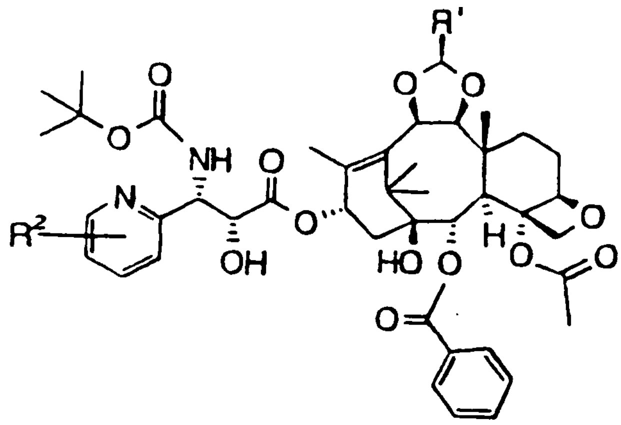

(worin $R^1$ für eine Dimethylaminomethylgruppe oder Morpholinomethylgruppe steht und $R^2$ für ein Halogenatom oder eine Alkoxygruppe mit 1 bis 6 Kohlenstoffatomen steht) oder ein Salz davon.

2. Verbindung oder ein Salz davon nach Anspruch 1, wobei $R^2$ für eine Methoxygruppe oder ein Fluoratom steht.

3. Verbindung nach Anspruch 1 der folgenden Formel

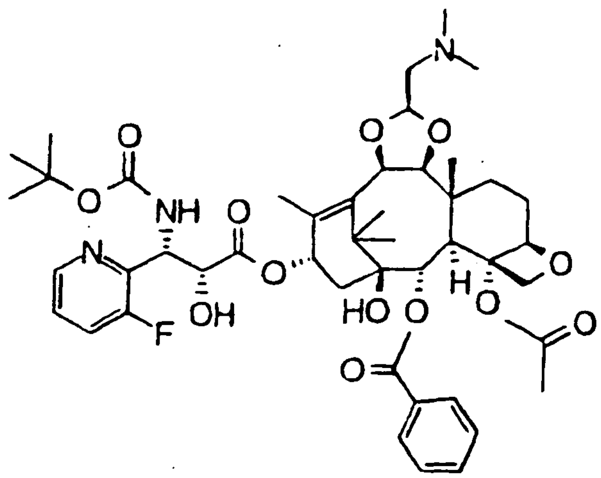

oder ein Salz davon.

4. Medikament, das eine in einem der Ansprüche 1 bis 3 beschriebene Verbindung oder ein Salz davon umfasst.

5. Antitumormittel, das eine in einem der Ansprüche 1 bis 3 beschriebene Verbindung oder ein Salz davon enthält.

6. Verbindung der folgenden Formel

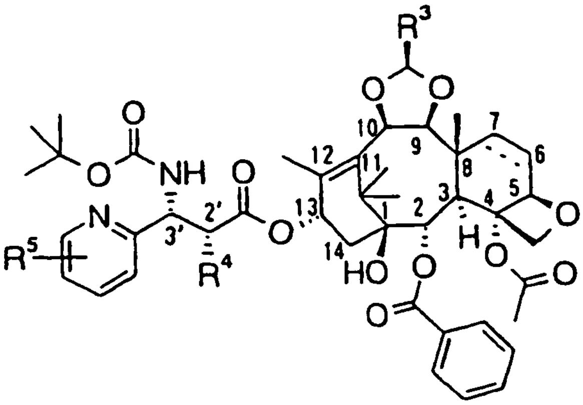

(worin $R^3$ für eine Dimethylaminomethylgruppe, Morpholinomethylgruppe oder Vinylgruppe steht, $R^4$ für eine Hydroxylgruppe, die eine Schutzgruppe aufweisen kann, steht und $R^5$ für ein Halogenatom oder eine Alkoxygruppe mit 1 bis 6 Kohlenstoffatomen steht und der Teil der gestrichelten Linie zwischen der 6-Position und 7-Position der Teilstruktur der Formel

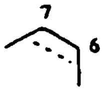

bedeutet, dass die Bindung des erwähnten Teils eine Doppelbindung sein kann (oder ein Salz davon).

**7.** Verbindung nach Anspruch 6 der folgenden Formel

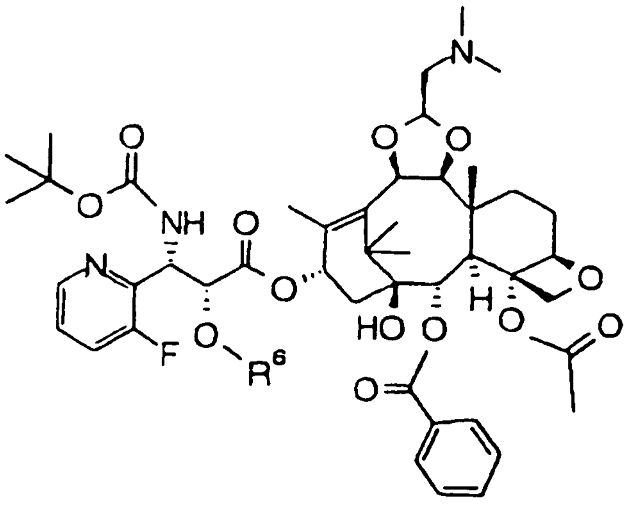

(worin $R^6$ eine Triisopropylsilylgruppe, tertiäre-Butyldimethylsilylgruppe, Triethylsilylgruppe oder Benzylgruppe bedeutet) oder ein Salz davon.

**8.** Verbindung nach Anspruch 6 der folgenden Formel

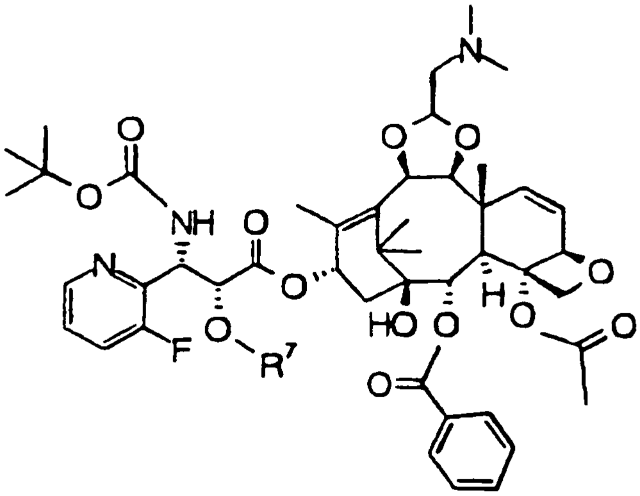

(worin $R^7$ eine Triisopropylsilylgruppe, tertiäre-Butyldimethylsilylgruppe, Triethylsilylgruppe oder Benzylgruppe bedeutet) oder ein Salz davon.

**9.** Verbindung nach Anspruch 6 der folgenden Formel:

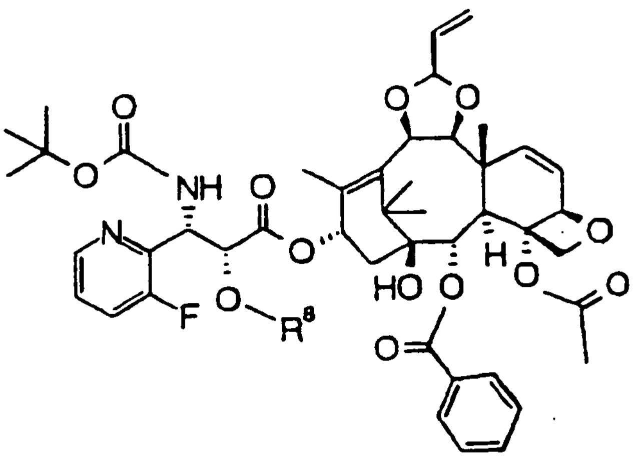

(worin $R^8$ eine Triisopropylsilylgruppe, tertiäre-Butyldimethylsilylgruppe, Triethylsilylgruppe oder Benzylgruppe bedeutet) oder ein Salz davon.

**10.** Verwendung einer in einem der Ansprüche 6 bis 9 beschriebenen Verbindung oder eines Salzes davon zur Herstellung einer in einem der Ansprüche 1 bis 3 beschriebenen Verbindung oder eines Salzes davon.

**11.** Verwendung nach Anspruch 10 einer Verbindung der folgenden Formel

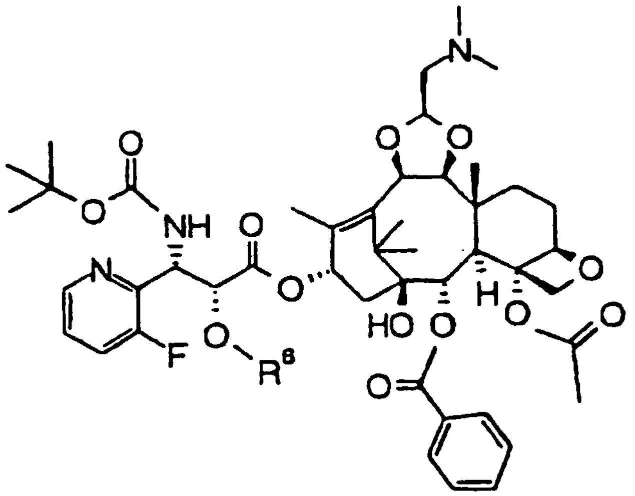

(worin $R^6$ eine Triisopropylsilylgruppe, tertiäre-Butyldimethylsilylgruppe, Triethylsilylgruppe oder Benzylgruppe bedeutet) oder eines Salzes davon, zur Herstellung einer Verbindung der folgenden Formel

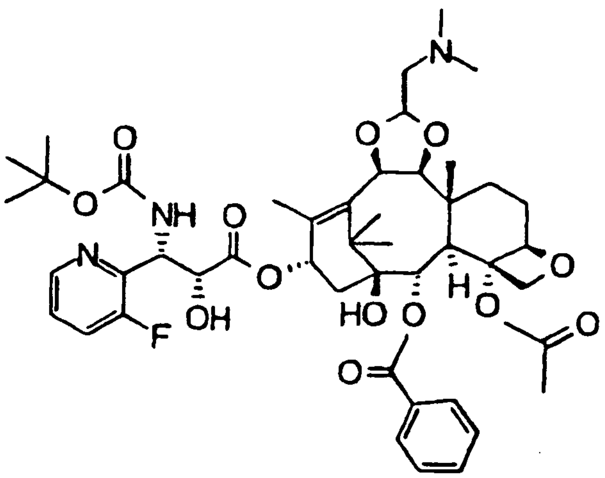

oder eines Salzes davon.

**12.** Verwendung nach Anspruch 10 einer Verbindung der folgenden Formel

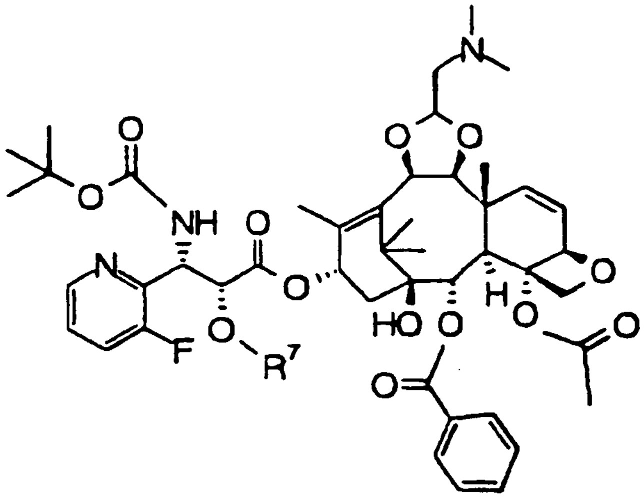

(worin $R^7$ eine Triisopropylsilylgruppe, tertiäre-Butyldimethylsilylgruppe, Triethylsilylgruppe oder Benzylgruppe bedeutet) oder eines Salzes davon, zur Herstellung einer Verbindung der folgenden Formel

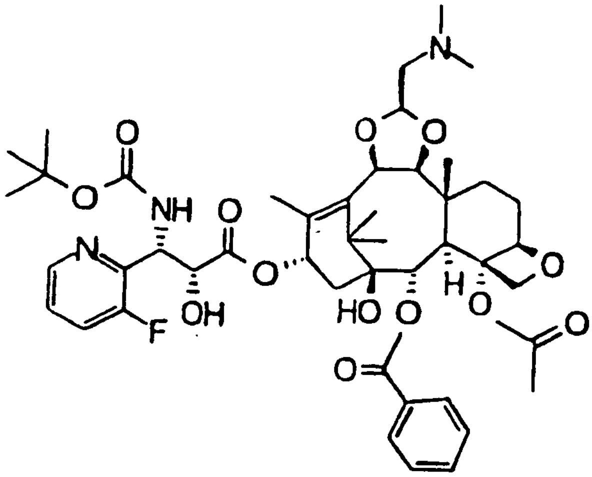

oder eines Salzes davon.

**13.** Verwendung nach Anspruch 10 einer Verbindung der folgenden Formel

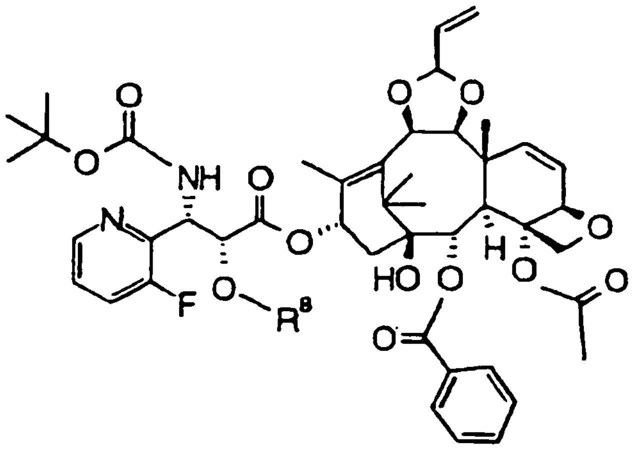

(worin $R^8$ eine Triisopropylsilylgruppe, tertiäre-Butyldimethylsilylgruppe, Triethylsilylgruppe oder Benzylgruppe bedeutet) oder eines Salzes davon, zur Herstellung einer Verbindung der folgenden Formel

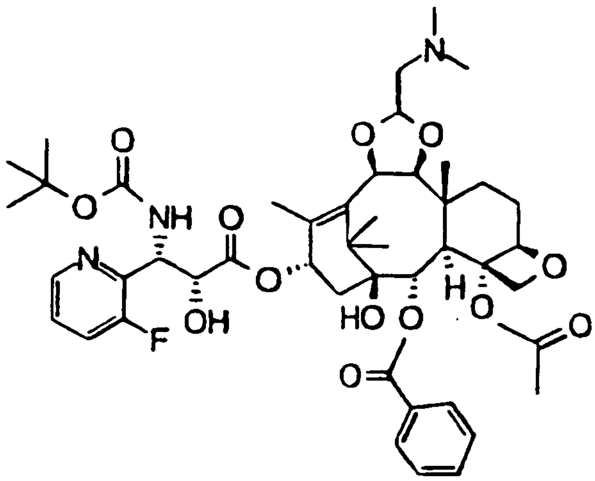

oder eines Salzes davon.

## Revendications

1. Composé représenté par la formule suivante

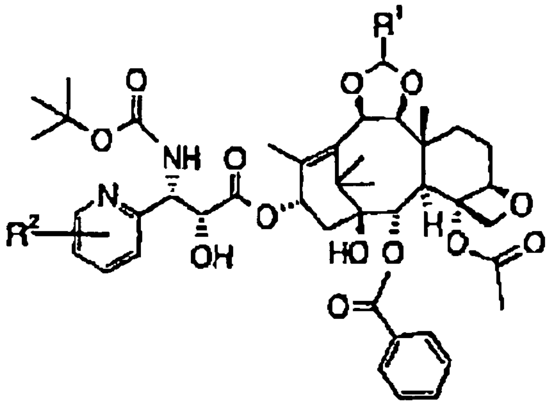

(dans laquelle $R^1$ représente un groupe diméthylaminométhyle ou un groupe morpholinométhyle et $R^2$ représente un atome d'halogène ou un groupe alcoxy ayant 1 à 6 atomes de carbone) ou un sel de celui-ci.

2. Composé ou sel de celui-ci selon la revendication 1, dans lequel $R^2$ est un groupe méthoxy ou un atome de fluor.

3. Composé selon la revendication 1, représenté par la formule suivante

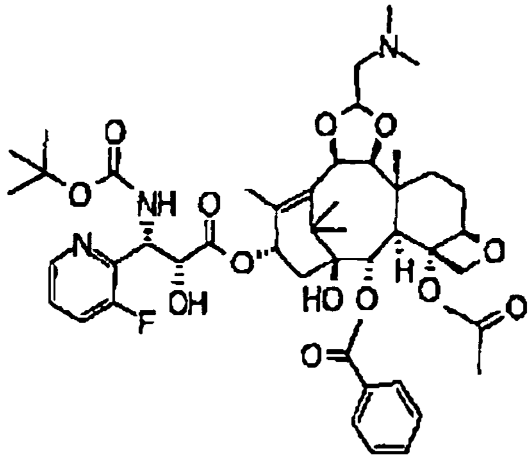

ou un sel de celui-ci.

4. Médicament qui comprend un composé ou un sel de celui-ci décrit selon l'une quelconque des revendications 1 à 3.

5. Agent anti-tumoral qui contient un composé ou un sel de celui-ci décrit selon l'une quelconque des revendications 1 à 3.

6. Composé représenté par la formule suivante

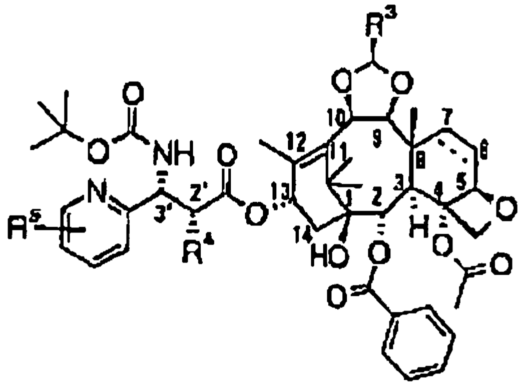

(dans laquelle $R^3$ représente un groupe diméthylaminométhyle, un groupe morpholinométhyle ou un groupe vinyle, $R^4$ représente un groupe hydroxyle qui peut avoir un groupe protecteur et $R^5$ représente un atome d'halogène ou un groupe alcoxy ayant 1 à 6 atomes de carbone, et la partie de ligne pointillée entre la position 6 et la position 7 d'une structure partielle montrée par la formule suivante

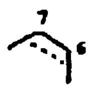

signifie que la liaison de ladite partie peut devenir une double liaison) ou un sel de celui-ci.

**7.** Composé selon la revendication 6, représenté par la formule suivante

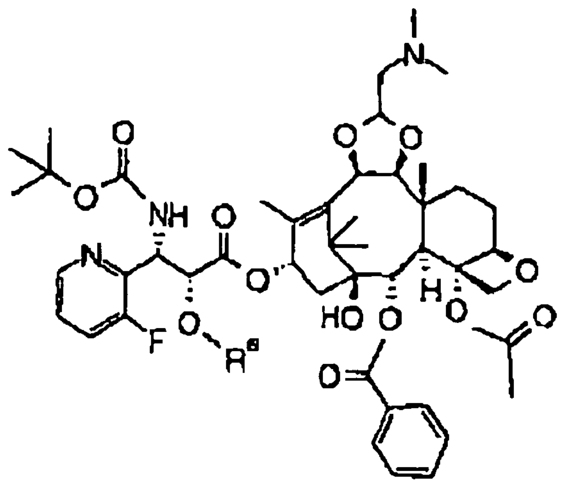

(dans laquelle $R^6$ représente un groupe triisopropylsilyle, un groupe butyldiméthylsilyle tertiaire, un groupe triéthylsilyle ou un groupe benzyle) ou un sel de celui-ci.

**8.** Composé selon la revendication 6, représenté par la formule suivante

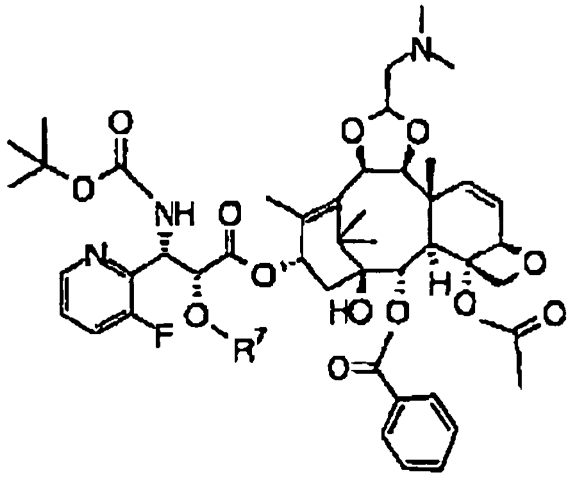

(dans laquelle $R^7$ représente un groupe triisopropylsilyle, un groupe butyldiméthylsilyle tertiaire, un groupe triéthylsilyle ou un groupe benzyle) ou un sel de celui-ci.

**9.** Composé selon la revendication 6, représenté par la formule suivante

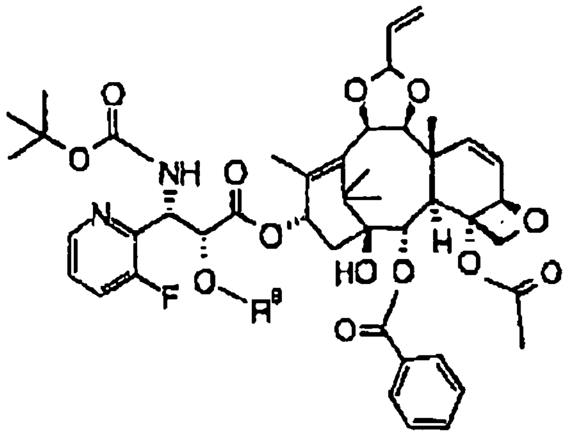

(dans laquelle $R^8$ représente un groupe triisopropylsilyle, un groupe butyldiméthylsilyle tertiaire, un groupe triéthylsilyle ou un groupe benzyle) ou un sel de celui-ci.

**10.** Utilisation d'un composé ou sel de celui-ci décrit selon l'une quelconque des revendications 6 à 9 pour produire un composé ou un sel de celui-ci décrit selon l'une quelconque des revendications 1 à 3.

**11.** Utilisation selon la revendication 10 d'un composé représenté par la formule suivante :

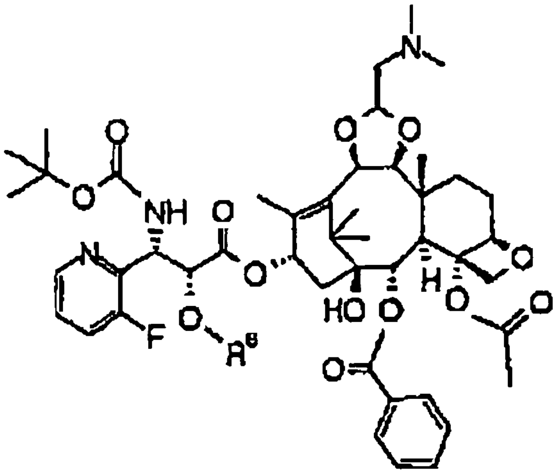

(dans laquelle $R^6$ représente un groupe triisopropylsilyle, un groupe butyldiméthylsilyle tertiaire, un groupe triéthylsilyle ou un groupe benzyle) ou un sel de celui-ci, pour produire un composé représenté par la formule suivante

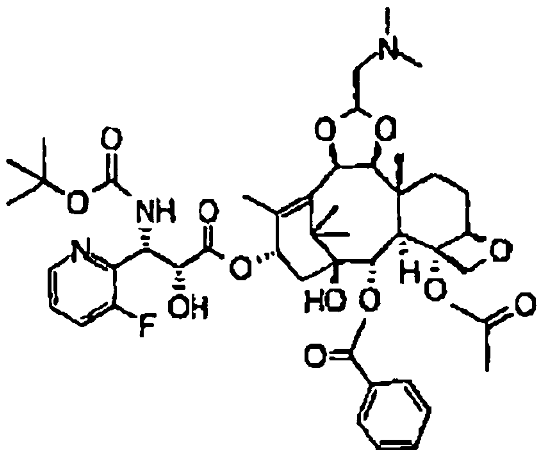

ou un sel de celui-ci.

**12.** Utilisation selon la revendication 10 d'un composé représenté par la formule suivante

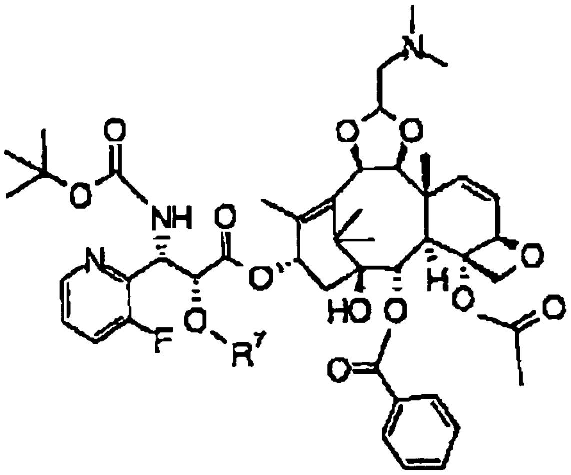

(dans laquelle $R^7$ représente un groupe triisopropylsilyle, un groupe butyldiméthylsilyle tertiaire, un groupe triéthylsilyle ou un groupe benzyle) ou un sel de celui-ci, pour produire un composé représenté par la formule suivante

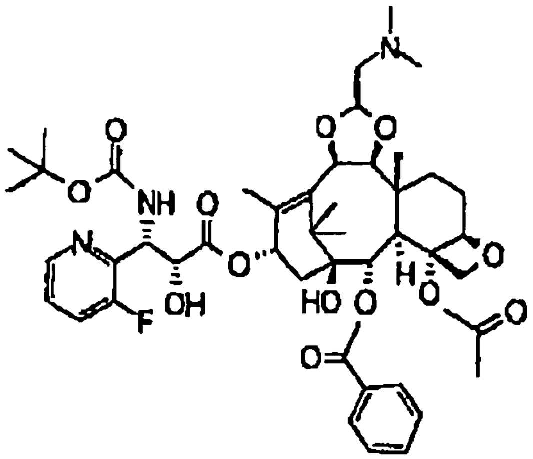

ou un sel de celui-ci.

**13.** Utilisation selon la revendication 10 d'un composé représenté par la formule suivante

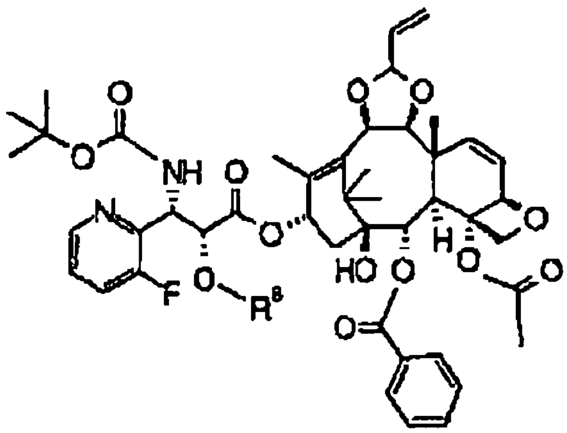

dans laquelle $R^8$ représente un groupe triisopropylsilyle, un groupe butyldiméthylsilyle tertiaire, un groupe triéthylsilyle ou un groupe benzyle) ou un sel de celui-ci, pour produire un composé représenté par la formule suivante

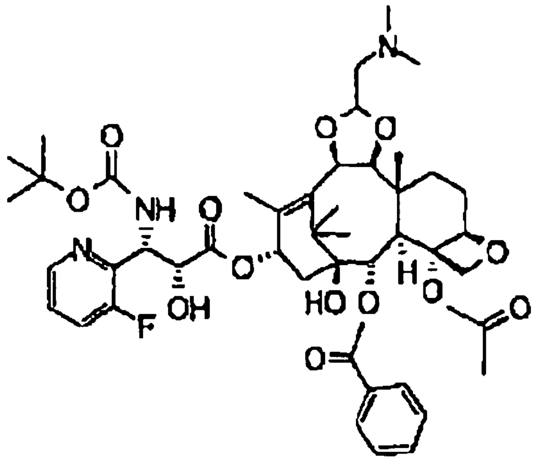

ou un sel de celui-ci.

FIG. 1

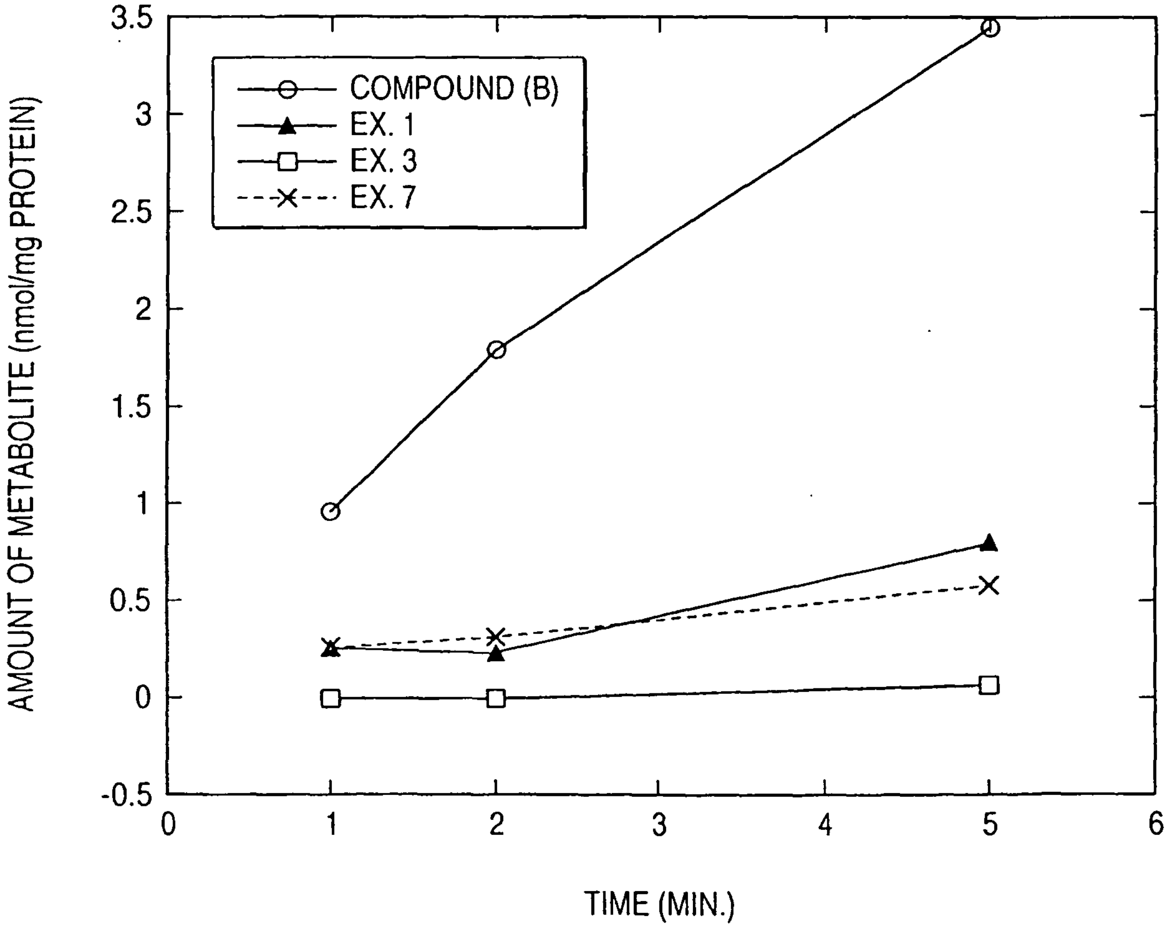
AMOUNT OF METABOLITE (nmol/mg PROTEIN)
3.5
3
2.5
2
1.5
1
0.5
0
-0.5
0
1
2
3
4
5
6
TIME (MIN.)
COMPOUND (B)
EX. 1
EX. 3
EX. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9012578 A


### Non-patent literature cited in the description


- *J. Am. Chem. Soc.,* 1988, vol. 110, 1917
- *Pharm. Tech. Japan,* 1997, vol. 13, 17-39
- *J. Pharmacol. Exp. Ther.,* 1997, vol. 283, 46-58
- Pharmacokinetic Studies on Drug Development. Yakuji Jiho, 15
- *Clin. Pharmacol. Ther.,* 1996, vol. 60, 601-607
- **KIYOSHI YAMAOKA ; YUSUKE TANIGAWARA.** *Yakubutsu Sokudoron Nyumon,* 116-117